# EUROPEAN PATENT APPLICATION

(11) **EP 1 557 468 A2**
(43) Date of publication of application: **27.07.2005**
(21) Application number: 03798037.2
(22) Date of filing: 15.09.2003
(51) Int. Cl.: C12N 15/82, A01H 1/00, A01H 5/00

(54) **VECTOR FOR THE PRODUCTION OF TRANSPLASTOMIC ANGIOSPERM PLANTS**

(30) Priority: 27.09.2002 CU 20802
(71) Applicant: CENTRO DE INGENIERIA GENETICA Y BIOTECNOLOGIA, Ciudad de La Habana 10600 (CU)
(72) Inventor: SELMAN-HOUSEIN SOSA, Guillermo, 12100 Ciudad de la Habana (CU); AGUIAR CABEZA, Eduardo, 64270 Sancti Spiritus (CU); GONZALEZ QUINTERO, Annery, del Carmen, 60200 Sancti Spiritus. (CU); RAMOS GONZALEZ, Osmany, 13500 Ciudad de la Habana (CU)
(74) Representative: Winckels, J.H.F., Mr. Ir.
(86) International application number: PCT/CU2003/000009
(87) International publication number: WO 2004/029256

(57) **Abstract**

A DNA vector is provided for stable insertion and expression of heterologous genes in the plastid genomes of any Angiosperm plant cell. In this vector, foreign genes can be inserted in multiple cloning sites located at an artificial intergenic region obtained from the combination of atpB and rbcL operons that belong to plants of different Classes (dicotyledonous and monocotyledonous). The expression cassette is inserted into the plastid genome by homologous recombination between the atpB and rbcL border sequences of the vector with the corresponding homologous regions of plastid genome; in such a way that more than one gene of interest can be expressed under the transcriptional control of the rbcL promoter adjacent to the border region that encodes for the atpB gene.

SEQUENCE LISTING

<110> Center for Genetic Engineering and Biotechnology.

<120> VECTOR FOR PRODUCTION OF ANGIOSPERM TRANSPLASTOMIC PLANTS.

<130> Vector for plastid transformation

<140> 0000
<141> 2002-08-05

<160> 26

<170> PatentIn Ver. 2.1

<210> 1
<211> 21
<212> DNA
<213> Artificial Sequence

<220>
<223> Description of Artificial Sequence: Oligonucleotide
      corresponding to the region -291 to -270 (from the
      start of translation) of the tobacco rbcL gene.

<400> 1
gggaagttct tattatttag g                                            21


<210> 2
<211> 20
<212> DNA
<213> Artificial Sequence

<220>
<223> Description of Artificial Sequence: Oligonucleotide
      corresponding to the region +1213 to +1233 (from the
      start of translation) of the tobacco rbcL gene.

<400> 2
ccaaggatgt cctaaagttc                                              20


<210> 3
<211> 133
<212> DNA
<213> Artificial Sequence

<220>
<223> Description of Artificial Sequence: Synthetic DNA
      fragment corresponding to the region -162 to -29
      of the non-translating lider of the tobacco rbcL
      gene, with modifications to introduce an "ideal"
      lacO.

<400> 3
ccatggtcta ataatcaaac attctgatta gttgataatt caaattgtga gcgctcacaa 60
tttgaaagat tcctgtgaaa agtttcatta acacggaatt cgtgtcgagt agaccttgtt 120
gttgtgagaa ttc                                                     133

<210> 4
<211> 1523
<212> DNA
<213> Artificial Sequence

<220>
<223> Description of Artificial Sequence: Nucleotide sequence
      of the rbcL-border.

<400> 4
gggaagttct tattatttag gttagtcagg tatttccatt tcaaaaaaaa aaaaagtaaa 60
aaagaaaaat tgggttgcgc tatatatatg aaagagtata caataatgat gtatttggca 120
aatcaaatac catggtctaa taatcaaaca ttctgattag ttgataattc aaattgtgag 180
cgctcacaat ttgaaagatt cctgtgaaaa gtttcattaa cacggaattc gtgtcgagta 240
gaccttgttg ttgtgagaat tcttaattca tgagttgtag ggagggattt atgtcaccac 300
aaacagagac taaagcaagt gttggattca aagctggtgt taaagagtac aaattgactt 360
attatactcc tgagtaccaa accaaggata ctgatatatt ggcagcattc cgagtaactc 420
ctcaacctgg agttccacct gaagaagcag gggccgcggt agctgccgaa tcttctactg 480
gtacatggac aactgtatgg accgatggac ttaccagcct tgatcgttac aaagggcgat 540
gctaccgcat cgagcgtgtt gttggagaaa aagatcaata tattgcttat gtagcttacc 600
ctttagacct ttttgaagaa ggttctgtta ccaacatgtt tacttccatt gtaggtaacg 660
tatttgggtt caaagccctg cgcgctctac gtctggaaga tctgcgaatc cctcctgctt 720
atgttaaaac tttccaaggt ccgcctcatg ggatccaagt tgaaagagat aaattgaaca 780
agtatggtcg tcccctgttg ggatgtacta ttaaacctaa attggggtta tctgctaaaa 840
actacggtag agccgtttat gaatgtcttc gcggtggact tgattttact aaagatgatg 900
agaacgtgaa ctcacaacca tttatgcgtt ggagagatcg tttcttattt tgtgccgaag 960
cactttataa agcacaggct gaaacaggtg aaatcaaagg gcattacttg aatgctactg 1020
caggtacatg cgaagaaatg atcaaaagag ctgtatttgc tagagaattg ggcgttccga 1080
tcgtaatgca tgactactta acggggggat tcaccgcaaa tactagcttg gctcattatt 1140
gccgagataa tggtctactt cttcacatcc accgtgcaat gcatgcggtt attgatagac 1200
agaagaatca tggtatccac ttccgggtat tagcaaaagc gttacgtatg tctggtggag 1260
atcatattca ctctggtacc gtagtaggta aacttgaagg tgaaagagac ataactttgg 1320
gctttgttga tttactgcgt gatgattttg ttgaacaaga tcgaagtcgc ggtatttatt 1380
tcactcaaga ttgggtctct ttaccaggtg ttctacccgt ggcttcagga ggtattcacg 1440
tttggcatat gcctgctctg accgagatct ttggggatga ttccgtacta cagttcggtg 1500
gaggaacttt aggacatcct tgg 1523

<210> 5
<211> 24
<212> DNA
<213> Artificial Sequence

<220>
<223> Description of Artificial Sequence: Oligonucleotide
      corresponding to the region -543 to -519 (from the
      start of translation) of the rice atpB gene.

<400> 5
gacttgagtt gttgttattg taag 24

<210> 6
<211> 23
<212> DNA
<213> Artificial Sequence

<220>
<223> Description of Artificial Sequence: Oligonucleotide

corresponding to the region +1188 to +1211 (from the
start of translation) of the rice atpB gene.

<400> 6
atgtcctgaa gttctttgta acg                                          23

<210> 7
<211> 132
<212> DNA
<213> Artificial Sequence

<220>
<223> Description of Artificial Sequence: Synthetic DNA
      fragment corresponding to the region -654 to -543
      of the non-translating lider of the rice atpB gene,
      with added restriction sites.

<400> 7
aagcttggcc aaaaaggccg tcgacaaaat ggggggcatg cttaagttaa tgaatatgtt 60
tcattcatat aatatgtttc attcatatat aatgggtaca ccctgtgtac attctatgct 120
ataggaattc at                                                      132

<210> 8
<211> 1887
<212> DNA
<213> Artificial Sequence

<220>
<223> Description of Artificial Sequence: Nucleotide sequence
      of the atpB-border.

<400> 8
aagcttggcc aaaaaggccg tcgacaaaat ggggggcatg cttaagttaa tgaatatgtt 60
tcattcatat aatatgtttc attcatatat aatgggtaca ccctgtgtac attctatgct 120
ataggaattc attcgacttg agttgttgtt attgtaagtt aacatgcttc gattattaaa 180
ccatggattt gattcaccaa atccatcttt attgtatact ctttaataga tatagcgcaa 240
ccccaaatca acttctaatc cttattaagt tcttaataga cccccttttc ttattttgag 300
tggaaatacc taaatactac gaaaattctc tgttgacagc aatctatgct tcacagtagt 360
atatattttg tatatcgaag tcctagataa gaaagtagag taggcacaaa tcgtttacaa 420
aaggcaaaat gtatatgaaa aaaagattga ttgaactttc cgacggactc attccatgag 480
taaacgattg aatgggattc gtttgggcaa cgaaatcaag tgctggtccc cttttctctc 540
ttattgaatt aactaattca tttccttttg acttttggat ttttggatat ttttttggtg 600
ttgatttggc attattcaac aagaaaaaaa tcaaaatttc gataaattcc ttttttttga 660
aaattatgtg ataattatga gaaccaatcc tactacttct cgtcccgggg tttctacaat 720
tgaagaaaaa agtacagggc gtatcgatca aattattgga cccgtgctgg atgtcacttt 780
tcccccgggc aagttacctt atatttataa tgctttggta gtcaagagtc gagacactga 840
cggtaagcaa attaatgtaa cttgtgaggt acaacaatta ttaggaaata atcgagttag 900
agctgtagct atgagtgcta cagatgggtt gatgagagga atggaagtga ttgacacggg 960
agctcctctc agtgttcctg tcggtggagc tactcttgga cgaattttca acgttcttgg 1020
ggagcctgtt gacaatttgg gtcctgtaga tactagtgca acattcccta ttcatagatc 1080
cgcgcccgcc tttatcgagt tagatacgaa attatccatc tttgaaactg gtattaaggt 1140
ggtcgatctt ttagctcctt atcggcgtgg aggaaaaatc ggactatttg ggggagctgg 1200
agtaggtaaa acagtactca tcatggaatt aatcaacaat attgctaaag ctcacggggg 1260
cgtatccgta tttggcggag taggggaacg gactcgtgaa ggaaatgatc tttatatgga 1320
aatgaaggaa tctggagtaa ttaatgaaaa aaatcttgag gaatcaaagg tagctctagt 1380
ctatggccaa atgaatgaac cgccaggagc tcgtatgaga gttggtttga ctgccctaac 1440
tatggcagaa tatttccgag atgttaataa gcaagacgtg cttctattca tcgataatat 1500
ctttcgtttt gttcaagcag gatcggaggt atctgcctta ttagggagaa tgccctctgc 1560
agtgggttat caacctactc ttagtacaga aatgggttct ttgcaagaaa gaattacttc 1620

tactaaaaag ggatctataa cttcgatcca agcggtttat gtacctgcgg acgatttgac 1680
cgaccctgct cctgctacaa catttgcaca tttggatgct actaccgtac tttccagagg 1740
attagcttcc aaagggattt atcctgcagt agatccttta gattcaacct caactatgtt 1800
acaacctcgg atcgttggca acgaacatta tgaaactgca caaagagtta agcaaacttt 1860
acaacgttac aaagaacttc aggacat 1887

<210> 9
<211> 24
<212> DNA
<213> Artificial Sequence

<220>
<223> Description of Artificial Sequence: Oligonucleotide primer
      used to amplify the hgh gene by PCR, with a RBS.

<400> 9
gggaggaatg agatatgaaa aagc 24

<210> 10
<211> 24
<212> DNA
<213> Artificial Sequence

<220>
<223> Description of Artificial Sequence: Oligonucleotide primer
      used to amplify the hgh gene by PCR, with added KpnI site.

<400> 10
gtcggtacct actctatttc tttg 24

<210> 11
<211> 41
<212> DNA
<213> Artificial Sequence

<220>
<223> Description of Artificial Sequence: Synthetic DNA
      fragment with useful restriction sites for the construction
      of the selection cassette with the hgh gene.

<400> 11
aagcttgatt cgagtgaacg cgtatagggc ccgggagatc t 41

<210> 12
<211> 2223
<212> DNA
<213> Artificial Sequence

<220>
<223> Description of Artificial Sequence: Nucleotide sequence of
      the DNA fragment containing the selection cassette with
      repeated borders that was cloned in the pBluescript.

<400> 12
aagcttgatt cgagtgaacg cgtatagggc ccaattcgag ctcggtacca gcaccaccag 60
cggtgaggtg cggaacttct acaacctcaa agcccataac gttgcggata gaacccttct 120
cagggtcaat cagagcagcg tagtttgctg cgttcggcat cagtgctgcc agaatcgcag 180

agtagctatc tgggtcacag tagaacacac ggtcagcagc cggaacatag ttcttggtca 240
gagccgcacg agccttagtc agagccgcaa taatctcctt acccagcgca acttggtcgg 300
tcaagtgcgg ccttgttctg agtggtctca attacggtag cagtacctaa gccctcgggg 360
gatctgggga ggaatgagat atgaaaaagc ctgaactcac cgcgacgtct gtcgagaagt 420
ttctgatcga aaagttcgac agcgtctccg acctgatgca gctctcggag ggcgaagaat 480
ctcgtgcttt cagcttcgat gtaggagggc gtggatatgt cctgcgggta aatagctgcg 540
ccgatggttt ctacaaagat cgttatgttt atcggcactt tgcatcggcc gcgctcccga 600
ttccggaagt gcttgacatt ggggagttta gcgagagcct gacctattgc atctcccgcc 660
gtgcacaggg tgtcacgttg caagacctgc ctgaaaccga actgcccgct gttctacaac 720
cggtcgcgga ggctatggat gcgatcgctg cggccgatct tagccagacg agcgggttcg 780
gcccattcgg accgcaagga atcggtcaat acactacatg gcgtgatttc atatgcgcga 840
ttgctgatcc ccatgtgtat cactggcaaa ctgtgatgga cgacaccgtc agtgcgtccg 900
tcgcgcaggc tctcgatgag ctgatgcttt gggccgagga ctgccccgaa gtccggcacc 960
tcgtgcacgc ggatttcggc tccaacaatg tcctgacgga caatggccgc ataacagcgg 1020
tcattgactg gagcgaggcg atgttcgggg attcccaata cgaggtcgcc aacatcttct 1080
tctggaggcc gtggttggct tgtatggagc agcagacgcg ctacttcgag cggaggcatc 1140
cggagcttgc aggatcgcca cgactccggg cgtatatgct ccgcattggt cttgaccaac 1200
tctatcagag cttggttgac ggcaatttcg atgatgcagc ttgggcgcag ggtcgatgcg 1260
acgcaatcgt ccgatccgga gccgggactg tcgggcgtac acaaatcgcc cgcagaagcg 1320
cggccgtctg gaccgatggc tgtgtagaag tactcgccga tagtggaaac cgacgcccca 1380
gcactcgtcc gagggcaaag aaatagagta ggtaccagca ccaccagcgg tgaggtgcgg 1440
aacttctaca acctcaaagc ccataacgtt gcggatagaa cccttctcag ggtcaatcag 1500
agcagcgtag tttgctgcgt tcggcatcag tgctgccaga atcgcagagt agctatctgg 1560
gtcacagtag aacacacggt cagcagccgg aacatagttc ttggtcagag ccgcacgagc 1620
cttagtcaga gccgcaataa tctccttacc cagcgcaact tggtcggtaa gtgcggcctt 1680
gttctgagtg gtctcaatta cggtagcagt acctaagccc tcgggggatc agcttggctg 1740
ttttggcgga tgagagaaga ttttcagcct gatacagatt aaatcagaac gcagaagcgg 1800
tctgataaaa cagaatttgc ctggcggcag tagcgcggtg gtcccacctg accccatgcc 1860
gaactcagaa gtgaaacgcc gtagcgccga tggtagtgtg gggtctcccc atgcgagagt 1920
agggaactgc caggcatcaa ataaaacgaa aggctcagtc gaaagactgg gcctttcgtt 1980
ttatctgttg tttgtcggtg aacgctctcc tgagtaggac aaatccgccg ggagcggatt 2040
tgaacgttgc gaagcaacgg cccggagggt ggcgggcagg acgcccgcca taaactgcca 2100
ggcatcaaat taagcagaag gccatcctga cggatggcct ttttgcgttt ctacaaactc 2160
tttttgttta tttttctaaa tacattcaaa tatgtatccg ctgggggatc cactagttct 2220
aga 2223

<210> 13
<211> 5669
<212> DNA
<213> Artificial Sequence

<220>
<223> Description of Artificial Sequence: Nucleotide sequence of DNA
      fragment from the vector pVTPA-f between the rice atpB and
      tobacco rbcL borders.

<400> 13
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780

gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgatt cgagtgaacg cgtatagggc 1920
ccaattcgag ctcggtacca gcaccaccag cggtgaggtg cggaacttct acaacctcaa 1980
agcccataac gttgcggata gaacccttct cagggtcaat cagagcagcg tagtttgctg 2040
cgttcggcat cagtgctgcc agaatcgcag agtagctatc tgggtcacag tagaacacac 2100
ggtcagcagc cggaacatag ttcttggtca gagccgcacg agccttagtc agagccgcaa 2160
taatctcctt acccagcgca acttggtcgg tcaagtgcgg ccttgttctg agtggtctca 2220
attacggtag cagtacctaa gccctcgggg gatctgggga ggaatgagat atgaaaaagc 2280
ctgaactcac cgcgacgtct gtcgagaagt ttctgatcga aaagttcgac agcgtctccg 2340
acctgatgca gctctcggag ggcgaagaat ctcgtgcttt cagcttcgat gtaggagggc 2400
gtggatatgt cctgcgggta aatagctgcg ccgatggttt ctacaaagat cgttatgttt 2460
atcggcactt tgcatcggcc gcgctcccga ttccggaagt gcttgacatt ggggagttta 2520
gcgagagcct gacctattgc atctcccgcc gtgcacaggg tgtcacgttg caagacctgc 2580
ctgaaaccga actgcccgct gttctacaac cggtcgcgga ggctatggat gcgatcgctg 2640
cggccgatct tagccagacg agcgggttcg gcccattcgg accgcaagga atcggtcaat 2700
acactacatg gcgtgatttc atatgcgcga ttgctgatcc ccatgtgtat cactggcaaa 2760
ctgtgatgga cgacaccgtc agtgcgtccg tcgcgcaggc tctcgatgag ctgatgcttt 2820
gggccgagga ctgccccgaa gtccggcacc tcgtgcacgc ggatttcggc tccaacaatg 2880
tcctgacgga caatggccgc ataacagcgg tcattgactg gagcgaggcg atgttcgggg 2940
attcccaata cgaggtcgcc aacatcttct tctggaggcc gtggttggct tgtatggagc 3000
agcagacgcg ctacttcgag cggaggcatc cggagcttgc aggatcgcca cgactccggg 3060
cgtatatgct ccgcattggt cttgaccaac tctatcagag cttggttgac ggcaatttcg 3120
atgatgcagc ttgggcgcag ggtcgatgcg acgcaatcgt ccgatccgga gccgggactg 3180
tcgggcgtac acaaatcgcc cgcagaagcg cggccgtctg gaccgatggc tgtgtagaag 3240
tactcgccga tagtggaaac cgacgcccca gcactcgtcc gagggcaaag aaatagagta 3300
ggtaccagca ccaccagcgg tgaggtgcgg aacttctaca acctcaaagc ccataacgtt 3360
gcggatagaa cccttctcag ggtcaatcag agcagcgtag tttgctgcgt tcggcatcag 3420
tgctgccaga atcgcagagt agctatctgg gtcacagtag aacacacggt cagcagccgg 3480
aacatagttc ttggtcagag ccgcacgagc cttagtcaga gccgcaataa tctccttacc 3540
cagcgcaact tggtcggtaa gtgcggcctt gttctgagtg gtctcaatta cggtagcagt 3600
acctaagccc tcgggggatc agcttggctg ttttggcgga tgagagaaga ttttcagcct 3660
gatacagatt aaatcagaac gcagaagcgg tctgataaaa cagaatttgc ctggcggcag 3720
tagcgcggtg gtcccacctg accccatgcc gaactcagaa gtgaaacgcc gtagcgccga 3780
tggtagtgtg gggtctcccc atgcgagagt agggaactgc caggcatcaa ataaaacgaa 3840
aggctcagtc gaaagactgg gcctttcgtt ttatctgttg tttgtcggtg aacgctctcc 3900
tgagtaggac aaatccgccg ggagcggatt tgaacgttgc gaagcaacgg cccggagggt 3960
ggcgggcagg acgcccgcca taaactgcca ggcatcaaat taagcagaag gccatcctga 4020
cggatggcct ttttgcgttt ctacaaactc tttttgttta tttttctaaa tacattcaaa 4080
tatgtatccg ctgggggatc agcttgatgg ccgccagtgt gatggatggg aagttcttat 4140
tatttaggtt agtcaggtat ttccatttca aaaaaaaaaa aagtaaaaaa gaaaaattgg 4200
gttgcgctat atatatgaaa gagtatacaa taatgatgta tttggcaaat caaataccat 4260
ggtctaataa tcaaacattc tgattagttg ataattcaaa ttgtgagcgc tcacaatttg 4320
aaagattcct gtgaaaagtt tcattaacac ggaattcgtg tcgagtagac cttgttgttg 4380
tgagaattct taattcatga gttgtaggga gggatttatg tcaccacaaa cagagactaa 4440

agcaagtgtt ggattcaaag ctggtgttaa agagtacaaa ttgacttatt atactcctga 4500
gtaccaaacc aaggatactg atatattggc agcattccga gtaactcctc aacctggagt 4560
tccacctgaa gaagcagggg ccgcggtagc tgccgaatct tctactggta catggacaac 4620
tgtatggacc gatggactta ccagccttga tcgttacaaa gggcgatgct accgcatcga 4680
gcgtgttgtt ggagaaaaag atcaatatat tgcttatgta gcttaccctt tagacctttt 4740
tgaagaaggt tctgttacca acatgtttac ttccattgta ggtaacgtat ttgggttcaa 4800
agccctgcgc gctctacgtc tggaagatct gcgaatccct cctgcttatg ttaaaacttt 4860
ccaaggtccg cctcatggga tccaagttga aagagataaa ttgaacaagt atggtcgtcc 4920
cctgttggga tgtactatta aacctaaatt ggggttatct gctaaaaact acggtagagc 4980
cgtttatgaa tgtcttcgcg gtggacttga ttttactaaa gatgatgaga acgtgaactc 5040
acaaccattt atgcgttgga gagatcgttt cttattttgt gccgaagcac tttataaagc 5100
acaggctgaa acaggtgaaa tcaaagggca ttacttgaat gctactgcag gtacatgcga 5160
agaaatgatc aaaagagctg tatttgctag agaattgggc gttccgatcg taatgcatga 5220
ctacttaacg gggggattca ccgcaaatac tagcttggct cattattgcc gagataatgg 5280
tctacttctt cacatccacc gtgcaatgca tgcggttatt gatagacaga agaatcatgg 5340
tatccacttc cgggtattag caaaagcgtt acgtatgtct ggtggagatc atattcactc 5400
tggtaccgta gtaggtaaac ttgaaggtga aagagacata actttgggct ttgttgattt 5460
actgcgtgat gattttgttg aacaagatcg aagtcgcggt atttatttca ctcaagattg 5520
ggtctcttta ccaggtgttc tacccgtggc ttcaggaggt attcacgttt ggcatatgcc 5580
tgctctgacc gagatctttg gggatgattc cgtactacag ttcggtggag gaactttagg 5640
acatccttgg atctgcagct agttctaga 5669

<210> 14
<211> 176
<212> DNA
<213> Artificial Sequence

<220>
<223> Description of Artificial Sequence: Synthetic DNA
      fragment codifying for the promoter region of the plastid 16S
      ribosomal RNA (Prrn), with added restriction sites.

<400> 14
gaattccccc gggctgctcc cccgccgtcg ttcaatgaga atggataaga ggctcgtggg 60
attgacgtga gggggcaggg atggctatat ttctgggagc gaactccggg cgaatacgaa 120
gcgcttggat acagttgtag ggagggattt catcgtttaa actcgagtga acgcgt 176

<210> 15
<211> 5834
<212> DNA
<213> Artificial Sequence

<220>
<223> Description of Artificial Sequence: Nucleotide sequence of DNA
      fragment from the vector pVTPA between the rice atpB and
      tobacco rbcL borders.

<400> 15
gtcgaggtca tgtcctgaag ttctttgtaa cgttgtaaag tttgcttaac tctttgtgca 60
gtttcataat g[?][?]cgt[?][?]cc aacgatccga ggt[?]gtaaca tagttgaggt tgaatctaaa 120
ggatctactg caggat[?]aat ccctttggaa gctaatcctc tggaaagtac ggtagtagca 180
tccaaatgtg caaatg[?][?]gt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660

ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgata tcgaattccc ccgggctgct 1920
cccccgccgt cgttcaatga gaatggataa gaggctcgtg ggattgacgt gagggggcag 1980
ggatggctat atttctggga gcgaactccg ggcgaatacg aagcgcttgg atacagttgt 2040
agggagggat ttcatcgttt aaactcgagt gaacgcgtat agggcccaat tcgagctcgg 2100
taccagcacc accagcggtg aggtgcggaa cttctacaac ctcaaagccc ataacgttgc 2160
ggatagaacc cttctcaggg tcaatcagag cagcgtagtt tgctgcgttc ggcatcagtg 2220
ctgccagaat cgcagagtag ctatctgggt cacagtagaa cacacggtca gcagccggaa 2280
catagttctt ggtcagagcc gcacgagcct tagtcagagc cgcaataatc tccttaccca 2340
gcgcaacttg gtcggtcaag tgcggccttg ttctgagtgg tctcaattac ggtagcagta 2400
cctaagccct cgggggatct ggggaggaat gagatatgaa aaagcctgaa ctcaccgcga 2460
cgtctgtcga gaagtttctg atcgaaaagt tcgacagcgt ctccgacctg atgcagctct 2520
cggagggcga agaatctcgt gctttcagct tcgatgtagg agggcgtgga tatgtcctgc 2580
gggtaaatag ctgcgccgat ggtttctaca aagatcgtta tgtttatcgg cactttgcat 2640
cggccgcgct cccgattccg gaagtgcttg acattgggga gtttagcgag agcctgacct 2700
attgcatctc ccgccgtgca cagggtgtca cgttgcaaga cctgcctgaa accgaactgc 2760
ccgctgttct acaaccggtc gcggaggcta tggatgcgat cgctgcggcc gatcttagcc 2820
agacgagcgg gttcggccca ttcggaccgc aaggaatcgg tcaatacact acatggcgtg 2880
atttcatatg cgcgattgct gatccccatg tgtatcactg gcaaactgtg atggacgaca 2940
ccgtcagtgc gtccgtcgcg caggctctcg atgagctgat gctttgggcc gaggactgcc 3000
ccgaagtccg gcacctcgtg cacgcggatt tcggctccaa caatgtcctg acggacaatg 3060
gccgcataac agcggtcatt gactggagcg aggcgatgtt cggggattcc caatacgagg 3120
tcgccaacat cttcttctgg aggccgtggt tggcttgtat ggagcagcag acgcgctact 3180
tcgagcggag gcatccggag cttgcaggat cgccacgact ccgggcgtat atgctccgca 3240
ttggtcttga ccaactctat cagagcttgg ttgacggcaa tttcgatgat gcagcttggg 3300
cgcagggtcg atgcgacgca atcgtccgat ccggagccgg gactgtcggg cgtacacaaa 3360
tcgcccgcag aagcgcggcc gtctggaccg atggctgtgt agaagtactc gccgatagtg 3420
gaaaccgacg ccccagcact cgtccgaggg caaagaaata gagtaggtac cagcaccacc 3480
agcggtgagg tgcggaactt ctacaacctc aaagcccata acgttgcgga tagaaccctt 3540
ctcagggtca atcagagcag cgtagtttgc tgcgttcggc atcagtgctg ccagaatcgc 3600
agagtagcta tctgggtcac agtagaacac acggtcagca gccggaacat agttcttggt 3660
cagagccgca cgagccttag tcagagccgc aataatctcc ttacccagcg caacttggtc 3720
ggtaagtgcg gccttgttct gagtggtctc aattacggta gcagtaccta agccctcggg 3780
ggatcagctt ggctgttttg gcggatgaga gaagattttc agcctgatac agattaaatc 3840
agaacgcaga agcggtctga taaaacagaa tttgcctggc ggcagtagcg cggtggtccc 3900
acctgacccc atgccgaact cagaagtgaa acgccgtagc gccgatggta gtgtggggtc 3960
tccccatgcg agagtaggga actgccaggc atcaaataaa acgaaaggct cagtcgaaag 4020
actgggcctt tcgttttatc tgttgtttgt cggtgaacgc tctcctgagt aggacaaatc 4080
cgccgggagc ggatttgaac gttgcgaagc aacggcccgg agggtggcgg gcaggacgcc 4140
cgccataaac tgccaggcat caaattaagc agaaggccat cctgacggat ggcctttttg 4200
cgtttctaca aactcttttt gtttattttt ctaaatacat tcaaatatgt atccgctggg 4260
ggatcagctt gatggccgcc agtgtgatgg atgggaagtt cttattattt aggttagtca 4320

ggtatttcca tttcaaaaaa aaaaaaagta aaaaagaaaa attgggttgc gctatatata 4380
tgaaagagta tacaataatg atgtatttgg caaatcaaat accatggtct aataatcaaa 4440
cattctgatt agttgataat tcaaattgtg agcgctcaca atttgaaaga ttcctgtgaa 4500
aagtttcatt aacacggaat tcgtgtcgag tagaccttgt tgttgtgaga attcttaatt 4560
catgagttgt agggagggat ttatgtcacc acaaacagag actaaagcaa gtgttggatt 4620
caaagctggt gttaaagagt acaaattgac ttattatact cctgagtacc aaaccaagga 4680
tactgatata ttggcagcat tccgagtaac tcctcaacct ggagttccac ctgaagaagc 4740
aggggccgcg gtagctgccg aatcttctac tggtacatgg acaactgtat ggaccgatgg 4800
acttaccagc cttgatcgtt acaaagggcg atgctaccgc atcgagcgtg ttgttggaga 4860
aaaagatcaa tatattgctt atgtagctta ccctttagac ctttttgaag aaggttctgt 4920
taccaacatg tttacttcca ttgtaggtaa cgtatttggg ttcaaagccc tgcgcgctct 4980
acgtctggaa gatctgcgaa tccctcctgc ttatgttaaa actttccaag gtccgcctca 5040
tgggatccaa gttgaaagag ataaattgaa caagtatggt cgtcccctgt tgggatgtac 5100
tattaaacct aaattggggt tatctgctaa aaactacggt agagccgttt atgaatgtct 5160
tcgcggtgga cttgatttta ctaaagatga tgagaacgtg aactcacaac catttatgcg 5220
ttggagagat cgtttcttat tttgtgccga agcactttat aaagcacagg ctgaaacagg 5280
tgaaatcaaa gggcattact tgaatgctac tgcaggtaca tgcgaagaaa tgatcaaaag 5340
agctgtattt gctagagaat tgggcgttcc gatcgtaatg catgactact taacgggggg 5400
attcaccgca aatactagct tggctcatta ttgccgagat aatggtctac ttcttcacat 5460
ccaccgtgca atgcatgcgg ttattgatag acagaagaat catggtatcc acttccgggt 5520
attagcaaaa gcgttacgta tgtctggtgg agatcatatt cactctggta ccgtagtagg 5580
taaacttgaa ggtgaaagag acataacttt gggctttgtt gatttactgc gtgatgattt 5640
tgttgaacaa gatcgaagtc gcggtattta tttcactcaa gattgggtct ctttaccagg 5700
tgttctaccc gtggcttcag gaggtattca cgtttggcat atgcctgctc tgaccgagat 5760
ctttggggat gattccgtac tacagttcgg tggaggaact ttaggacatc cttggatctg 5820
cagctagttc taga 5834

<210> 16
<211> 96
<212> DNA
<213> Artificial Sequence

<220>
<223> Description of Artificial Sequence: Nucleotide sequence of the
      chimeric PpsbA* promoter with added restriction sites and a RBS.

<400> 16
gaattcacct tggttgacac gagtatataa gtcatgttat actgttgaat aaaaagcctt 60
ccattttgat taaataaagg aggattttca tatgat 96

<210> 17
<211> 106
<212> DNA
<213> Artificial Sequence

<220>
<223> Description of Artificial Sequence: Synthetic DNA fragment
      codifying for a mini-cistron, a RBS, and restriction sites.

<400> 17
catatgtatc gattacgtaa ggaggaataa accatggacg agctctagac tgcagcatgc 60
ccgggatcct aggcctgata tcaagcttct cgagctgtcg acagct 106

<210> 18
<211> 365
<212> DNA
<213> Artificial Sequence

<220>
<223> Description of Artificial Sequence: Nucleotide sequence of DNA
      fragment from the vector pVIEP showing the expression cassette
      and surrounding restriction sites.

<400> 18
ggtaccgggc cccccctcga ggtcgacggt atcgataagc ttgatatcga attcaccttg 60
gttgacacga gtatataagt catgttatac tgttgaataa aaagccttcc attttgatta 120
aataaaggag gattttcata tgtatcgatt acgtaaggag gaataaacca tggacgagct 180
ctagactgca gcatgcccgg gatccggctg ctaacaaagc ccgaaaggaa gctgagttgg 240
ctgctgccac cgctgagcaa taactagcat aaccccttgg ggcctctaaa cgggtcttga 300
ggggtttttt gctgaaagga ggaactatat ccggatcctg atatcaagct tctcgagctg 360
tcgac                                                             365

<210> 19
<211> 7510
<212> DNA
<213> Artificial Sequence

<220>
<223> Description of Artificial Sequence: Nucleotide sequence of DNA
      fragment from the vector pVTPA-f-GUS between the rice atpB and
      tobacco rbcL borders.

<400> 19
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct cccatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa agcaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgcctttttgt aaacgatttg tgcctactct 1500
actttcttat ctaggacttc gatatacaaa atatatacta ctgtgaagca tagattgctg 1560
tcaacagaga atttccgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgata tcggtaagga ggaataaacc 1920
atggtacgtc ctgtagaaac cccaacccgt gaaatcaaaa aactcgacgg cctgtgggca 1980
ttcagtctgg atcgcgaaaa ctgtggaatt gatcagcgtt ggtgggaaag cgcgttacaa 2040
gaaagccggg caattgctgt gccaggcagt tttaacgatc agttcgccga tgcagatatt 2100

cgtaattatg cgggcaacgt ctggtatcag cgcgaagtct ttataccgaa aggttgggca 2160
ggccagcgta tcgtgctgcg tttcgatgcg gtcactcatt acggcaaagt gtgggtcaat 2220
aatcaggaag tgatggagca tcagggcggc tatacgccat ttgaagccga tgtcacgccg 2280
tatgttattg ccgggaaaag tgtacgtatc accgtttgtg tgaacaacga actgaactgg 2340
cagactatcc cgccgggaat ggtgattacc gacgaaaacg gcaagaaaaa gcagtcttac 2400
ttccatgatt tctttaacta tgccggaatc catcgcagcg taatgctcta caccacgccg 2460
aacacctggg tggacgatat caccgtggtg acgcatgtcg cgcaagactg taaccacgcg 2520
tctgttgact ggcaggtggt ggccaatggt gatgtcagcg ttgaactgcg tgatgcggat 2580
caacaggtgg ttgcaactgg acaaggcact agcgggactt tgcaagtggt gaatccgcac 2640
ctctggcaac cgggtgaagg ttatctctat gaactgtgcg tcacagccaa aagccagaca 2700
gagtgtgata tctacccgct tcgcgtcggc atccggtcag tggcagtgaa gggcgaacag 2760
ttcctgatta accacaaacc gttctacttt actggctttg gtcgtcatga agatgcggac 2820
ttgcgtggca aaggattcga taacgtgctg atggtgcacg accacgcatt aatggactgg 2880
attggggcca actcctaccg tacctcgcat tacccttacg ctgaagagat gctcgactgg 2940
gcagatgaac atggcatcgt ggtgattgat gaaactgctg ctgtcggctt taacctctct 3000
ttaggcattg gtttcgaagc gggcaacaag ccgaaagaac tgtacagcga agaggcagtc 3060
aacggggaaa ctcagcaagc gcacttacag gcgattaaag agctgatagc gcgtgacaaa 3120
aaccacccaa gcgtggtgat gtggagtatt gccaacgaac cggatacccg tccgcaaggt 3180
gcacgggaat atttcgcgcc actggcggaa gcaacgcgta aactcgaccc gacgcgtccg 3240
atcacctgcg tcaatgtaat gttctgcgac gctcacaccg ataccatcag cgatctcttt 3300
gatgtgctgt gcctgaaccg ttattacgga tggtatgtcc aaagcggcga tttggaaacg 3360
gcagagaagg tactggaaaa agaacttctg gcctggcagg agaaactgca tcagccgatt 3420
atcatcaccg aatacggcgt ggatacgtta gccgggctgc actcaatgta caccgacatg 3480
tggagtgaag agtatcagtg tgcatggctg gatatgtatc accgcgtctt tgatcgcgtc 3540
agcgccgtcg tcggtgaaca ggtatggaat ttcgccgatt ttgcgacctc gcaaggcata 3600
ttgcgcgttg gcggtaacaa gaaagggatc ttcactcgcg accgcaaacc gaagtcggcg 3660
gcttttctgc tgcaaaaacg ctggactggc atgaacttcg gtgaaaaacc gcagcaggga 3720
ggcaaacaat gaattcgagc tcggtacccc gcgtataggg cccaattcga gctcggtacc 3780
agcaccacca gcggtgaggt gcggaacttc tacaacctca aagcccataa cgttgcggat 3840
agaacccttc tcagggtcaa tcagagcagc gtagtttgct gcgttcggca tcagtgctgc 3900
cagaatcgca gagtagctat ctgggtcaca gtagaacaca cggtcagcag ccggaacata 3960
gttcttggtc agagccgcac gagccttagt cagagccgca ataatctcct tacccagcgc 4020
aacttggtcg gtcaagtgcg gccttgttct gagtggtctc aattacggta gcagtaccta 4080
agccctcggg ggatctgggg aggaatgaga tatgaaaaag cctgaactca ccgcgacgtc 4140
tgtcgagaag tttctgatcg aaaagttcga cagcgtctcc gacctgatgc agctctcgga 4200
gggcgaagaa tctcgtgctt tcagcttcga tgtaggaggg cgtggatatg tcctgcgggt 4260
aaatagctgc gccgatggtt tctacaaaga tcgttatgtt tatcggcact ttgcatcggc 4320
cgcgctcccg attccggaag tgcttgacat tggggagttt agcgagagcc tgacctattg 4380
catctcccgc cgtgcacagg gtgtcacgtt gcaagacctg cctgaaaccg aactgcccgc 4440
tgttctacaa ccggtcgcgg aggctatgga tgcgatcgct gcggccgatc ttagccagac 4500
gagcgggttc ggcccattcg gaccgcaagg aatcggtcaa tacactacat ggcgtgattt 4560
catatgcgcg attgctgatc cccatgtgta tcactggcaa actgtgatgg acgacaccgt 4620
cagtgcgtcc gtcgcgcagg ctctcgatga gctgatgctt tgggccgagg actgccccga 4680
agtccggcac ctcgtgcacg cggatttcgg ctccaacaat gtcctgacgg acaatggccg 4740
cataacagcg gtcattgact ggagcgaggc gatgttcggg gattcccaat acgaggtcgc 4800
caacatcttc ttctggaggc cgtggttggc ttgtatggag cagcagacgc gctacttcga 4860
gcggaggcat ccggagcttg caggatcgcc acgactccgg gcgtatatgc tccgcattgg 4920
tcttgaccaa ctctatcaga gcttggttga cggcaatttc gatgatgcag cttgggcgca 4980
gggtcgatgc gacgcaatcg tccgatccgg agccgggact gtcgggcgta cacaaatcgc 5040
ccgcagaagc gcggccgtct ggaccgatgg ctgtgtagaa gtactcgccg atagtggaaa 5100
ccgacgcccc agcactcgtc cgagggcaaa gaaatagagt aggtaccagc accaccagcg 5160
gtgaggtgcg gaacttctac aacctcaaag cccataacgt tgcggataga acccttctca 5220
gggtcaatca gagcagcgta gtttgctgcg ttcggcatca gtgctgccag aatcgcagag 5280
tagctatctg ggtcacagta gaacacacgg tcagcagccg gaacatagtt cttggtcaga 5340
gccgcacgag ccttagtcag agccgcaata atctccttac ccagcgcaac ttggtcggta 5400
agtgcggcct tgttctgagt ggtctcaatt acggtagcag tacctaagcc ctcgggggat 5460
cagcttggct gttttggcgg atgagagaag attttcagcc tgatacagat taaatcagaa 5520
cgcagaagcg gtctgataaa acagaatttg cctggcggca gtagcgcggt ggtcccacct 5580
gaccccatgc cgaactcaga agtgaaacgc cgtagcgccg atggtagtgt ggggtctccc 5640
catgcgagag tagggaactg ccaggcatca aataaaacga aaggctcagt cgaaagactg 5700
ggcctttcgt tttatctgtt gtttgtcggt gaacgctctc ctgagtagga caaatccgcc 5760

gggagcggat ttgaacgttg cgaagcaacg gcccggaggg tggcgggcag gacgcccgcc 5820
ataaactgcc aggcatcaaa ttaagcagaa ggccatcctg acggatggcc tttttgcgtt 5880
tctacaaact ctttttgttt atttttctaa atacattcaa atatgtatcc gctgggggat 5940
cagcttgatg gccgccagtg tgatggatgg gaagttctta ttatttaggt tagtcaggta 6000
tttccatttc aaaaaaaaaa aaagtaaaaa agaaaaattg ggttgcgcta tatatatgaa 6060
agagtataca ataatgatgt atttggcaaa tcaaatacca tggtctaata atcaaacatt 6120
ctgattagtt gataattcaa attgtgagcg ctcacaattt gaaagattcc tgtgaaaagt 6180
ttcattaaca cggaattcgt gtcgagtaga ccttgttgtt gtgagaattc ttaattcatg 6240
agttgtaggg agggatttat gtcaccacaa acagagacta aagcaagtgt tggattcaaa 6300
gctggtgtta aagagtacaa attgacttat tatactcctg agtaccaaac caaggatact 6360
gatatattgg cagcattccg agtaactcct caacctggag ttccacctga agaagcaggg 6420
gccgcggtag ctgccgaatc ttctactggt acatggacaa ctgtatggac cgatggactt 6480
accagccttg atcgttacaa agggcgatgc taccgcatcg agcgtgttgt tggagaaaaa 6540
gatcaatata ttgcttatgt agcttaccct ttagaccttt ttgaagaagg ttctgttacc 6600
aacatgttta cttccattgt aggtaacgta tttgggttca aagccctgcg cgctctacgt 6660
ctggaagatc tgcgaatccc tcctgcttat gttaaaactt tccaaggtcc gcctcatggg 6720
atccaagttg aaagagataa attgaacaag tatggtcgtc ccctgttggg atgtactatt 6780
aaacctaaat tggggttatc tgctaaaaac tacggtagag ccgtttatga atgtcttcgc 6840
ggtggacttg attttactaa agatgatgag aacgtgaact cacaaccatt tatgcgttgg 6900
agagatcgtt tcttattttg tgccgaagca ctttataaag cacaggctga aacaggtgaa 6960
atcaaagggc attacttgaa tgctactgca ggtacatgcg aagaaatgat caaaagagct 7020
gtatttgcta gagaattggg cgttccgatc gtaatgcatg actacttaac ggggggattc 7080
accgcaaata ctagcttggc tcattattgc cgagataatg gtctacttct tcacatccac 7140
cgtgcaatgc atgcggttat tgatagacag aagaatcatg gtatccactt ccgggtatta 7200
gcaaaagcgt tacgtatgtc tggtggagat catattcact ctggtaccgt agtaggtaaa 7260
cttgaaggtg aaagagacat aactttgggc tttgttgatt tactgcgtga tgattttgtt 7320
gaacaagatc gaagtcgcgg tatttatttc actcaagatt gggtctcttt accaggtgtt 7380
ctacccgtgg cttcaggagg tattcacgtt tggcatatgc ctgctctgac cgagatcttt 7440
ggggatgatt ccgtactaca gttcggtgga ggaactttag gacatccttg gatctgcagc 7500
tagttctaga 7510

<210> 20
<211> 33
<212> DNA
<213> Artificial Sequence

<220>
<223> Description of Artificial Sequence: Oligonucleotide primer
      used to amplify the aadA gene by PCR, with a RBS.

<400> 20
aaggagaata ccatggggga agcggtgatc gcc 33

<210> 21
<211> 29
<212> DNA
<213> Artificial Sequence

<220>
<223> Description of Artificial Sequence: Oligonucleotide primer
      used to amplify the aadA gene by PCR.

<400> 21
gaacgagctc ttagacatta tttgccgac 29

<210> 22
<211> 6659
<212> DNA

<213> Artificial Sequence

<220>
<223> Description of Artificial Sequence: Nucleotide sequence of DNA
      fragment from the vector pVTPA-aadA between the rice atpB and
      tobacco rbcL borders.

<400> 22
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgata tcgaattccc ccgggctgct 1920
cccccgccgt cgttcaatga gaatggataa gaggctcgtg ggattgacgt gagggggcag 1980
ggatggctat atttctggga gcgaactccg ggcgaatacg aagcgcttgg atacagttgt 2040
agggagggat ttcatcgttt aaactcgaaa ggagaatacc atgggggaag cggtgatcgc 2100
cgaagtatcg actcaactat cagaggtagt tggcgtcatc gagcgccatc tcgaaccgac 2160
gttgctggcc gtacatttgt acggctccgc agtggatggc ggcctgaagc cacacagtga 2220
tattgatttg ctggttacgg tgaccgtaag gcttgatgaa acaacgcggc gagctttgat 2280
caacgacctt ttggaaactt cggcttcccc tggagagagc gagattctcc gcgctgtaga 2340
agtcaccatt gttgtgcacg acgacatcat tccgtggcgt tatccagcta agcgcgaact 2400
gcaatttgga gaatggcagc gcaatgacat tcttgcaggt atcttcgagc cagccacgat 2460
cgacattgat ctggctatct tgctgacaaa agcaagagaa catagcgttg ccttggtagg 2520
tccagcggcg gaggaactct ttgatccggt tcctgaacag gatctatttg aggcgctaaa 2580
tgaaacctta acgctatgga actcgccgcc cgactgggct ggcgatgagc gaaatgtagt 2640
gcttacgttg tcccgcattt ggtacagcgc agtaaccggc aaaatcgcgc cgaaggatgt 2700
cgctgccgac tgggcaatgg agcgcctgcc ggcccagtat cagcccgtca tacttgaagc 2760
tagacaggct tatcttggac aagaagaaga tcgcttggcc tcgcgcgcag atcagttgga 2820
agaatttgtc cactacgtga aaggcgagat caccaaggta gtcggcaaat aatgtctaag 2880
agctcgttct cgagtgaacg cgtatagggc ccaattcgag ctcggtacca gcaccaccag 2940
cggtgaggtg cggaacttct acaacctcaa agcccataac gttgcggata gaacccttct 3000
cagggtcaat cagagcagcg tagtttgctg cgttcggcat cagtgctgcc agaatcgcag 3060
agtagctatc tgggtcacag tagaacacac ggtcagcagc cggaacatag ttcttggtca 3120
gagccgcacg agccttagtc agagccgcaa taatctcctt acccagcgca acttggtcgg 3180

tcaagtgcgg ccttgttctg agtggtctca attacggtag cagtacctaa gccctcgggg 3240
gatctgggga ggaatgagat atgaaaaagc ctgaactcac cgcgacgtct gtcgagaagt 3300
ttctgatcga aaagttcgac agcgtctccg acctgatgca gctctcggag ggcgaagaat 3360
ctcgtgcttt cagcttcgat gtaggagggc gtggatatgt cctgcgggta aatagctgcg 3420
ccgatggttt ctacaaagat cgttatgttt atcggcactt tgcatcggcc gcgctcccga 3480
ttccggaagt gcttgacatt ggggagttta gcgagagcct gacctattgc atctcccgcc 3540
gtgcacaggg tgtcacgttg caagacctgc ctgaaaccga actgcccgct gttctacaac 3600
cggtcgcgga ggctatggat gcgatcgctg cggccgatct tagccagacg agcgggttcg 3660
gcccattcgg accgcaagga atcggtcaat acactacatg gcgtgatttc atatgcgcga 3720
ttgctgatcc ccatgtgtat cactggcaaa ctgtgatgga cgacaccgtc agtgcgtccg 3780
tcgcgcaggc tctcgatgag ctgatgcttt gggccgagga ctgccccgaa gtccggcacc 3840
tcgtgcacgc ggatttcggc tccaacaatg tcctgacgga caatggccgc ataacagcgg 3900
tcattgactg gagcgaggcg atgttcgggg attcccaata cgaggtcgcc aacatcttct 3960
tctggaggcc gtggttggct tgtatggagc agcagacgcg ctacttcgag cggaggcatc 4020
cggagcttgc aggatcgcca cgactccggg cgtatatgct ccgcattggt cttgaccaac 4080
tctatcagag cttggttgac ggcaatttcg atgatgcagc ttgggcgcag ggtcgatgcg 4140
acgcaatcgt ccgatccgga gccgggactg tcgggcgtac acaaatcgcc cgcagaagcg 4200
cggccgtctg gaccgatggc tgtgtagaag tactcgccga tagtggaaac cgacgcccca 4260
gcactcgtcc gagggcaaag aaatagagta ggtaccagca ccaccagcgg tgaggtgcgg 4320
aacttctaca acctcaaagc ccataacgtt gcggatagaa cccttctcag ggtcaatcag 4380
agcagcgtag tttgctgcgt tcggcatcag tgctgccaga atcgcagagt agctatctgg 4440
gtcacagtag aacacacggt cagcagccgg aacatagttc ttggtcagag ccgcacgagc 4500
cttagtcaga gccgcaataa tctccttacc cagcgcaact tggtcggtaa gtgcggcctt 4560
gttctgagtg gtctcaatta cggtagcagt acctaagccc tcgggggatc agcttggctg 4620
ttttggcgga tgagagaaga ttttcagcct gatacagatt aaatcagaac gcagaagcgg 4680
tctgataaaa cagaatttgc ctggcggcag tagcgcggtg gtcccacctg accccatgcc 4740
gaactcagaa gtgaaacgcc gtagcgccga tggtagtgtg gggtctcccc atgcgagagt 4800
agggaactgc caggcatcaa ataaaacgaa aggctcagtc gaaagactgg gcctttcgtt 4860
ttatctgttg tttgtcggtg aacgctctcc tgagtaggac aaatccgccg ggagcggatt 4920
tgaacgttgc gaagcaacgg cccggagggt ggcgggcagg acgcccgcca taaactgcca 4980
ggcatcaaat taagcagaag gccatcctga cggatggcct ttttgcgttt ctacaaactc 5040
tttttgttta tttttctaaa tacattcaaa tatgtatccg ctgggggatc agcttgatgg 5100
ccgccagtgt gatggatggg aagttcttat tatttaggtt agtcaggtat ttccatttca 5160
aaaaaaaaaa aagtaaaaaa gaaaaattgg gttgcgctat atatatgaaa gagtatacaa 5220
taatgatgta tttggcaaat caaataccat ggtctaataa tcaaacattc tgattagttg 5280
ataattcaaa ttgtgagcgc tcacaatttg aaagattcct gtgaaaagtt tcattaacac 5340
ggaattcgtg tcgagtagac cttgttgttg tgagaattct taattcatga gttgtaggga 5400
gggatttatg tcaccacaaa cagagactaa agcaagtgtt ggattcaaag ctggtgttaa 5460
agagtacaaa ttgacttatt atactcctga gtaccaaacc aaggatactg atatattggc 5520
agcattccga gtaactcctc aacctggagt tccacctgaa gaagcagggg ccgcggtagc 5580
tgccgaatct tctactggta catggacaac tgtatggacc gatggactta ccagccttga 5640
tcgttacaaa gggcgatgct accgcatcga gcgtgttgtt ggagaaaaag atcaatatat 5700
tgcttatgta gcttaccctt tagacctttt tgaagaaggt tctgttacca acatgtttac 5760
ttccattgta ggtaacgtat ttgggttcaa agccctgcgc gctctacgtc tggaagatct 5820
gcgaatccct cctgcttatg ttaaaacttt ccaaggtccg cctcatggga tccaagttga 5880
aagagataaa ttgaacaagt atggtcgtcc cctgttggga tgtactatta aacctaaatt 5940
ggggttatct gctaaaaact acggtagagc cgtttatgaa tgtcttcgcg gtggacttga 6000
ttttactaaa gatgatgaga acgtgaactc acaaccattt atgcgttgga gagatcgttt 6060
cttattttgt gccgaagcac tttataaagc acaggctgaa acaggtgaaa tcaaagggca 6120
ttacttgaat gctactgcag gtacatgcga agaaatgatc aaaagagctg tatttgctag 6180
agaattgggc gttccgatcg taatgcatga ctacttaacg gggggattca ccgcaaatac 6240
tagcttggct cattattgcc gagataatgg tctacttctt cacatccacc gtgcaatgca 6300
tgcggttatt gatagacaga agaatcatgg tatccacttc cgggtattag caaaagcgtt 6360
acgtatgtct ggtggagatc atattcactc tggtaccgta gtaggtaaac ttgaaggtga 6420
aagagacata actttgggct ttgttgattt actgcgtgat gattttgttg aacaagatcg 6480
aagtcgcggt atttatttca ctcaagattg ggtctcttta ccaggtgttc tacccgtggc 6540
ttcaggaggt attcacgttt ggcatatgcc tgctctgacc gagatctttg gggatgattc 6600
cgtactacag ttcggtggag gaactttagg acatccttgg atctgcagct agttctaga 6659

<210> 23

<211> 8327
<212> DNA
<213> Artificial Sequence

<220>
<223> Description of Artificial Sequence: Nucleotide sequence of DNA
      fragment from the vector pVTPA-f-GUS-aadA between the rice atpB
      and tobacco rbcL borders.

<400> 23
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgata tcggtaagga ggaataaacc 1920
atggtacgtc ctgtagaaac cccaacccgt gaaatcaaaa aactcgacgg cctgtgggca 1980
ttcagtctgg atcgcgaaaa ctgtggaatt gatcagcgtt ggtgggaaag cgcgttacaa 2040
gaaagccggg caattgctgt gccaggcagt tttaacgatc agttcgccga tgcagatatt 2100
cgtaattatg cgggcaacgt ctggtatcag cgcgaagtct ttataccgaa aggttgggca 2160
ggccagcgta tcgtgctgcg tttcgatgcg gtcactcatt acggcaaagt gtgggtcaat 2220
aatcaggaag tgatggagca tcagggcggc tatacgccat ttgaagccga tgtcacgccg 2280
tatgttattg ccgggaaaag tgtacgtatc accgtttgtg tgaacaacga actgaactgg 2340
cagactatcc cgccgggaat ggtgattacc gacgaaaacg gcaagaaaaa gcagtcttac 2400
ttccatgatt tctttaacta tgccggaatc catcgcagcg taatgctcta caccacgccg 2460
aacacctggg tggacgatat caccgtggtg acgcatgtcg cgcaagactg taaccacgcg 2520
tctgttgact ggcaggtggt ggccaatggt gatgtcagcg ttgaactgcg tgatgcggat 2580
caacaggtgg ttgcaactgg acaaggcact agcgggactt tgcaagtggt gaatccgcac 2640
ctctggcaac cgggtgaagg ttatctctat gaactgtgcg tcacagccaa aagccagaca 2700
gagtgtgata tctacccgct tcgcgtcggc atccggtcag tggcagtgaa gggcgaacag 2760
ttcctgatta accacaaacc gttctacttt actggctttg gtcgtcatga agatgcggac 2820
ttgcgtggca aaggattcga taacgtgctg atggtgcacg accacgcatt aatggactgg 2880
attggggcca actcctaccg tacctcgcat tacccttacg ctgaagagat gctcgactgg 2940
gcagatgaac atggcatcgt ggtgattgat gaaactgctg ctgtcggctt taacctctct 3000
ttaggcattg gtttcgaagc gggcaacaag ccgaaagaac tgtacagcga agaggcagtc 3060

aacggggaaa ctcagcaagc gcacttacag gcgattaaag agctgatagc gcgtgacaaa 3120
aaccacccaa gcgtggtgat gtggagtatt gccaacgaac cggatacccg tccgcaaggt 3180
gcacgggaat atttcgcgcc actggcggaa gcaacgcgta aactcgaccc gacgcgtccg 3240
atcacctgcg tcaatgtaat gttctgcgac gctcacaccg ataccatcag cgatctcttt 3300
gatgtgctgt gcctgaaccg ttattacgga tggtatgtcc aaagcggcga tttggaaacg 3360
gcagagaagg tactggaaaa agaacttctg gcctggcagg agaaactgca tcagccgatt 3420
atcatcaccg aatacggcgt ggatacgtta gccgggctgc actcaatgta caccgacatg 3480
tggagtgaag agtatcagtg tgcatggctg gatatgtatc accgcgtctt tgatcgcgtc 3540
agcgccgtcg tcggtgaaca ggtatggaat ttcgccgatt ttgcgacctc gcaaggcata 3600
ttgcgcgttg gcggtaacaa gaaagggatc ttcactcgcg accgcaaacc gaagtcggcg 3660
gcttttctgc tgcaaaaacg ctggactggc atgaacttcg gtgaaaaacc gcagcaggga 3720
ggcaaacaat gaattcgagc tcggtacccc gcgtatagaa ggagaatacc atgggggaag 3780
cggtgatcgc cgaagtatcg actcaactat cagaggtagt tggcgtcatc gagcgccatc 3840
tcgaaccgac gttgctggcc gtacatttgt acggctccgc agtggatggc ggcctgaagc 3900
cacacagtga tattgatttg ctggttacgg tgaccgtaag gcttgatgaa acaacgcggc 3960
gagctttgat caacgacctt ttggaaactt cggcttcccc tggagagagc gagattctcc 4020
gcgctgtaga agtcaccatt gttgtgcacg acgacatcat tccgtggcgt tatccagcta 4080
agcgcgaact gcaatttgga gaatggcagc gcaatgacat tcttgcaggt atcttcgagc 4140
cagccacgat cgacattgat ctggctatct tgctgacaaa agcaagagaa catagcgttg 4200
ccttggtagg tccagcggcg gaggaactct ttgatccggt tcctgaacag gatctatttg 4260
aggcgctaaa tgaaacctta acgctatgga actcgccgcc cgactgggct ggcgatgagc 4320
gaaatgtagt gcttacgttg tcccgcattt ggtacagcgc agtaaccggc aaaatcgcgc 4380
cgaaggatgt cgctgccgac tgggcaatgg agcgcctgcc ggcccagtat cagcccgtca 4440
tacttgaagc tagacaggct tatcttggac aagaagaaga tcgcttggcc tcgcgcgcag 4500
atcagttgga agaatttgtc cactacgtga aaggcgagat caccaaggta gtcggcaaat 4560
aatgtctaag agctcgttcc aattcgagct cggtaccagc accaccagcg gtgaggtgcg 4620
gaacttctac aacctcaaag cccataacgt tgcggataga acccttctca gggtcaatca 4680
gagcagcgta gtttgctgcg ttcggcatca gtgctgccag aatcgcagag tagctatctg 4740
ggtcacagta gaacacacgg tcagcagccg gaacatagtt cttggtcaga gccgcacgag 4800
ccttagtcag agccgcaata atctccttac ccagcgcaac ttggtcggtc aagtgcggcc 4860
ttgttctgag tggtctcaat tacggtagca gtacctaagc cctcggggga tctggggagg 4920
aatgagatat gaaaaagcct gaactcaccg cgacgtctgt cgagaagttt ctgatcgaaa 4980
agttcgacag cgtctccgac ctgatgcagc tctcggaggg cgaagaatct cgtgctttca 5040
gcttcgatgt aggagggcgt ggatatgtcc tgcgggtaaa tagctgcgcc gatggtttct 5100
acaaagatcg ttatgtttat cggcactttg catcggccgc gctcccgatt ccggaagtgc 5160
ttgacattgg ggagtttagc gagagcctga cctattgcat ctcccgccgt gcacagggtg 5220
tcacgttgca agacctgcct gaaaccgaac tgcccgctgt tctacaaccg gtcgcggagg 5280
ctatggatgc gatcgctgcg gccgatctta gccagacgag cgggttcggc ccattcggac 5340
cgcaaggaat cggtcaatac actacatggc gtgatttcat atgcgcgatt gctgatcccc 5400
atgtgtatca ctggcaaact gtgatggacg acaccgtcag tgcgtccgtc gcgcaggctc 5460
tcgatgagct gatgctttgg gccgaggact gccccgaagt ccggcacctc gtgcacgcgg 5520
atttcggctc caacaatgtc ctgacggaca atggccgcat aacagcggtc attgactgga 5580
gcgaggcgat gttcggggat tcccaatacg aggtcgccaa catcttcttc tggaggccgt 5640
ggttggcttg tatggagcag cagacgcgct acttcgagcg gaggcatccg gagcttgcag 5700
gatcgccacg actccgggcg tatatgctcc gcattggtct tgaccaactc tatcagagct 5760
tggttgacgg caatttcgat gatgcagctt gggcgcaggg tcgatgcgac gcaatcgtcc 5820
gatccggagc cgggactgtc gggcgtacac aaatcgcccg cagaagcgcg gccgtctgga 5880
ccgatggctg tgtagaagta ctcgccgata gtggaaaccg acgccccagc actcgtccga 5940
gggcaaagaa atagagtagg taccagcacc accagcggtg aggtgcggaa cttctacaac 6000
ctcaaagccc ataacgttgc ggatagaacc cttctcaggg tcaatcagag cagcgtagtt 6060
tgctgcgttc ggcatcagtg ctgccagaat cgcagagtag ctatctgggt cacagtagaa 6120
cacacggtca gcagccggaa catagttctt ggtcagagcc gcacgagcct tagtcagagc 6180
cgcaataatc tccttaccca gcgcaacttg gtcggtaagt gcggccttgt tctgagtggt 6240
ctcaattacg gtagcagtac ctaagccctc gggggatcag cttggctgtt ttggcggatg 6300
agagaagatt ttcagcctga tacagattaa atcagaacgc agaagcggtc tgataaaaca 6360
gaatttgcct ggcggcagta gcgcggtggt cccacctgac cccatgccga actcagaagt 6420
gaaacgccgt agcgccgatg gtagtgtggg gtctccccat gcgagagtag ggaactgcca 6480
ggcatcaaat aaaacgaaag gctcagtcga aagactgggc ctttcgtttt atctgttgtt 6540
tgtcggtgaa cgctctcctg agtaggacaa atccgccggg agcggatttg aacgttgcga 6600
agcaacggcc cggagggtgg cgggcaggac gcccgccata aactgccagg catcaaatta 6660
agcagaaggc catcctgacg gatggccttt ttgcgtttct acaaactctt tttgtttatt 6720

tttctaaata cattcaaata tgtatccgct gggggatcag cttgatggcc gccagtgtga 6780
tggatgggaa gttcttatta tttaggttag tcaggtattt ccatttcaaa aaaaaaaaaa 6840
gtaaaaaaga aaaattgggt tgcgctatat atatgaaaga gtatacaata atgatgtatt 6900
tggcaaatca aataccatgg tctaataatc aaacattctg attagttgat aattcaaatt 6960
gtgagcgctc acaatttgaa agattcctgt gaaaagtttc attaacacgg aattcgtgtc 7020
gagtagacct tgttgttgtg agaattctta attcatgagt tgtagggagg gatttatgtc 7080
accacaaaca gagactaaag caagtgttgg attcaaagct ggtgttaaag agtacaaatt 7140
gacttattat actcctgagt accaaaccaa ggatactgat atattggcag cattccgagt 7200
aactcctcaa cctggagttc cacctgaaga agcaggggcc gcggtagctg ccgaatcttc 7260
tactggtaca tggacaactg tatggaccga tggacttacc agccttgatc gttacaaagg 7320
gcgatgctac cgcatcgagc gtgttgttgg agaaaaagat caatatattg cttatgtagc 7380
ttacccttta gacctttttg aagaaggttc tgttaccaac atgtttactt ccattgtagg 7440
taacgtattt gggttcaaag ccctgcgcgc tctacgtctg gaagatctgc gaatccctcc 7500
tgcttatgtt aaaactttcc aaggtccgcc tcatgggatc caagttgaaa gagataaatt 7560
gaacaagtat ggtcgtcccc tgttgggatg tactattaaa cctaaattgg ggttatctgc 7620
taaaaactac ggtagagccg tttatgaatg tcttcgcggt ggacttgatt ttactaaaga 7680
tgatgagaac gtgaactcac aaccatttat gcgttggaga gatcgtttct tattttgtgc 7740
cgaagcactt tataaagcac aggctgaaac aggtgaaatc aaagggcatt acttgaatgc 7800
tactgcaggt acatgcgaag aaatgatcaa aagagctgta tttgctagag aattgggcgt 7860
tccgatcgta atgcatgact acttaacggg gggattcacc gcaaatacta gcttggctca 7920
ttattgccga gataatggtc tacttcttca catccaccgt gcaatgcatg cggttattga 7980
tagacagaag aatcatggta tccacttccg ggtattagca aaagcgttac gtatgtctgg 8040
tggagatcat attcactctg gtaccgtagt aggtaaactt gaaggtgaaa gagacataac 8100
tttgggcttt gttgatttac tgcgtgatga ttttgttgaa caagatcgaa gtcgcggtat 8160
ttatttcact caagattggg tctctttacc aggtgttcta cccgtggctt caggaggtat 8220
tcacgtttgg catatgcctg ctctgaccga gatctttggg gatgattccg tactacagtt 8280
cggtggagga actttaggac atccttggat ctgcagctag ttctaga 8327

<210> 24
<211> 7549
<212> DNA
<213> Artificial Sequence

<220>
<223> Description of Artificial Sequence: Nucleotide sequence of DNA
      fragment from the vector pVTPA-HB-aadA between the rice atpB and
      tobacco rbcL borders.

<400> 24
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260

ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acaggtgtta 1800
cccattatat atgaatgaaa catattatat gaatgaaaca tattcattaa cttaagcatg 1860
ccccccattt tgtcgaggtc gacggtatcg ataagcttga tatcggtaag gaggaataaa 1920
ccatggagaa catcacatca ggattcctag gacccctgct cgtgttacag gcggggtttt 1980
tcttgttgac aagaatcctc acaataccgc agagtctaga ctcgtggtgg acttctctca 2040
attttctagg gggatctccc gtgtgtcttg gccaaaattc gcagtcccca acctccaatc 2100
actcaccaac ctcctgtcct ccaatttgtc ctggttatcg ctggatgtgt ctgcggcgtt 2160
ttatcatatt cctcttcatc ctgctgctat gcctcatctt cttattggtt cttctggatt 2220
atcaaggtat gttgcccgtt tgtcctctaa ttccaggatc aacaacaacc agtacgggac 2280
catgcaaaac ctgcacgact cctgctcaag gcaactctat gtttccctca tgttgctgta 2340
caaaacctac ggatggaaat tgcacctgta ttcccatccc atcgtcctgg gctttcgcaa 2400
aatacctatg ggagtgggcc tcagtccgtt tctcttggct cagtttacta gtgccatttg 2460
ttcagtggtt cgtagggctt tcccccactg tttggctttc agctatatgg atgatgtggt 2520
attgggggcc aagtctgtac agcatcgtga gtccctttat accgctgtta ccaattttct 2580
tttgtctctg ggtatacatt taagaattgg gatccggctg ctaacaaagc ccgaaaggaa 2640
gctgagttgg ctgctgccac cgctgagcaa taactagcat aaccccttgg ggcctctaaa 2700
cgggtcttga ggggtttttt gctgaaagga ggaactatat ccggatcctg atatcaagct 2760
tctcgacggc ctttttggcc aagcttgata tcgaattccc ccgggctgct cccccgccgt 2820
cgttcaatga gaatggataa gaggctcgtg ggattgacgt gagggggcag ggatggctat 2880
atttctggga gcgaactccg ggcgaatacg aagcgcttgg atacagttgt agggagggat 2940
ttcatcgttt aaactcgaaa ggagaatacc atgggggaag cggtgatcgc cgaagtatcg 3000
actcaactat cagaggtagt tggcgtcatc gagcgccatc tcgaaccgac gttgctggcc 3060
gtacatttgt acggctccgc agtggatggc ggcctgaagc cacacagtga tattgatttg 3120
ctggttacgg tgaccgtaag gcttgatgaa acaacgcggc gagctttgat caacgacctt 3180
ttggaaactt cggcttcccc tggagagagc gagattctcc gcgctgtaga agtcaccatt 3240
gttgtgcacg acgacatcat tccgtggcgt tatccagcta agcgcgaact gcaatttgga 3300
gaatggcagc gcaatgacat tcttgcaggt atcttcgagc cagccacgat cgacattgat 3360
ctggctatct tgctgacaaa agcaagagaa catagcgttg ccttggtagg tccagcggcg 3420
gaggaactct ttgatccggt tcctgaacag gatctatttg aggcgctaaa tgaaacctta 3480
acgctatgga actcgccgcc cgactgggct ggcgatgagc gaaatgtagt gcttacgttg 3540
tcccgcattt ggtacagcgc agtaaccggc aaaatcgcgc cgaaggatgt cgctgccgac 3600
tgggcaatgg agcgcctgcc ggcccagtat cagcccgtca tacttgaagc tagacaggct 3660
tatcttggac aagaagaaga tcgcttggcc tcgcgcgcag atcagttgga agaatttgtc 3720
cactacgtga aaggcgagat caccaaggta gtcggcaaat aatgtctaag agctcgttct 3780
cgagtgaacg cgtatagggc ccaattcgag ctcggtacca gcaccaccag cggtgaggtg 3840
cggaacttct acaacctcaa agcccataac gttgcggata gaacccttct cagggtcaat 3900
cagagcagcg tagtttgctg cgttcggcat cagtgctgcc agaatcgcag agtagctatc 3960
tgggtcacag tagaacacac ggtcagcagc cggaacatag ttcttggtca gagccgcacg 4020
agccttagtc agagccgcaa taatctcctt acccagcgca acttggtcgg tcaagtgcgg 4080
ccttgttctg agtggtctca attacggtag cagtacctaa gccctcgggg gatctgggga 4140
ggaatgagat atgaaaaagc ctgaactcac cgcgacgtct gtcgagaagt ttctgatcga 4200
aaagttcgac agcgtctccg acctgatgca gctctcggag ggcgaagaat ctcgtgcttt 4260
cagcttcgat gtaggagggc gtggatatgt cctgcgggta aatagctgcg ccgatggttt 4320
ctacaaagat cgttatgttt atcggcactt tgcatcggcc gcgctcccga ttccggaagt 4380
gcttgacatt ggggagttta gcgagagcct gacctattgc atctcccgcc gtgcacaggg 4440
tgtcacgttg caagacctgc ctgaaaccga actgcccgct gttctacaac cggtcgcgga 4500
ggctatggat gcgatcgctg cggccgatct tagccagacg agcgggttcg gcccattcgg 4560
accgcaagga atcggtcaat acactacatg gcgtgatttc atatgcgcga ttgctgatcc 4620
ccatgtgtat cactggcaaa ctgtgatgga cgacaccgtc agtgcgtccg tcgcgcaggc 4680
tctcgatgag ctgatgcttt gggccgagga ctgccccgaa gtccggcacc tcgtgcacgc 4740
ggatttcggc tccaacaatg tcctgacgga caatggccgc ataacagcgg tcattgactg 4800
gagcgaggcg atgttcgggg attcccaata cgaggtcgcc aacatcttct tctggaggcc 4860
gtggttggct tgtatggagc agcagacgcg ctacttcgag cggaggcatc cggagcttgc 4920

aggatcgcca cgactccggg cgtatatgct ccgcattggt cttgaccaac tctatcagag 4980
cttggttgac ggcaatttcg atgatgcagc ttgggcgcag ggtcgatgcg acgcaatcgt 5040
ccgatccgga gccgggactg tcgggcgtac acaaatcgcc cgcagaagcg cggccgtctg 5100
gaccgatggc tgtgtagaag tactcgccga tagtggaaac cgacgcccca gcactcgtcc 5160
gagggcaaag aaatagagta ggtaccagca ccaccagcgg tgaggtgcgg aacttctaca 5220
acctcaaagc ccataacgtt gcggatagaa cccttctcag ggtcaatcag agcagcgtag 5280
tttgctgcgt tcggcatcag tgctgccaga atcgcagagt agctatctgg gtcacagtag 5340
aacacacggt cagcagccgg aacatagttc ttggtcagag ccgcacgagc cttagtcaga 5400
gccgcaataa tctccttacc cagcgcaact tggtcggtaa gtgcggcctt gttctgagtg 5460
gtctcaatta cggtagcagt acctaagccc tcgggggatc agcttggctg ttttggcgga 5520
tgagagaaga ttttcagcct gatacagatt aaatcagaac gcagaagcgg tctgataaaa 5580
cagaatttgc ctggcggcag tagcgcggtg gtcccacctg accccatgcc gaactcagaa 5640
gtgaaacgcc gtagcgccga tggtagtgtg gggtctcccc atgcgagagt agggaactgc 5700
caggcatcaa ataaaacgaa aggctcagtc gaaagactgg gcctttcgtt ttatctgttg 5760
tttgtcggtg aacgctctcc tgagtaggac aaatccgccg ggagcggatt tgaacgttgc 5820
gaagcaacgg cccggagggt ggcgggcagg acgcccgcca taaactgcca ggcatcaaat 5880
taagcagaag gccatcctga cggatggcct ttttgcgttt ctacaaactc tttttgttta 5940
tttttctaaa tacattcaaa tatgtatccg ctgggggatc agcttgatgg ccgccagtgt 6000
gatggatggg aagttcttat tatttaggtt agtcaggtat ttccatttca aaaaaaaaaa 6060
aagtaaaaaa gaaaaattgg gttgcgctat atatatgaaa gagtatacaa taatgatgta 6120
tttggcaaat caaataccat ggtctaataa tcaaacattc tgattagttg ataattcaaa 6180
ttgtgagcgc tcacaatttg aaagattcct gtgaaaagtt tcattaacac ggaattcgtg 6240
tcgagtagac cttgttgttg tgagaattct taattcatga gttgtaggga gggatttatg 6300
tcaccacaaa cagagactaa agcaagtgtt ggattcaaag ctggtgttaa agagtacaaa 6360
ttgacttatt atactcctga gtaccaaacc aaggatactg atatattggc agcattccga 6420
gtaactcctc aacctggagt tccacctgaa gaagcagggg ccgcggtagc tgccgaatct 6480
tctactggta catggacaac tgtatggacc gatggactta ccagccttga tcgttacaaa 6540
gggcgatgct accgcatcga gcgtgttgtt ggagaaaaag atcaatatat tgcttatgta 6600
gcttaccctt tagacctttt tgaagaaggt tctgttacca acatgtttac ttccattgta 6660
ggtaacgtat ttgggttcaa agccctgcgc gctctacgtc tggaagatct gcgaatccct 6720
cctgcttatg ttaaaacttt ccaaggtccg cctcatggga tccaagttga aagagataaa 6780
ttgaacaagt atggtcgtcc cctgttggga tgtactatta aacctaaatt ggggttatct 6840
gctaaaaact acggtagagc cgtttatgaa tgtcttcgcg gtggacttga ttttactaaa 6900
gatgatgaga acgtgaactc acaaccattt atgcgttgga gagatcgttt cttattttgt 6960
gccgaagcac tttataaagc acaggctgaa acaggtgaaa tcaaagggca ttacttgaat 7020
gctactgcag gtacatgcga agaaatgatc aaaagagctg tatttgctag agaattgggc 7080
gttccgatcg taatgcatga ctacttaacg gggggattca ccgcaaatac tagcttggct 7140
cattattgcc gagataatgg tctacttctt cacatccacc gtgcaatgca tgcggttatt 7200
gatagacaga agaatcatgg tatccacttc cgggtattag caaaagcgtt acgtatgtct 7260
ggtggagatc atattcactc tggtaccgta gtaggtaaac ttgaaggtga aagagacata 7320
actttgggct ttgttgattt actgcgtgat gattttgttg aacaagatcg aagtcgcggt 7380
atttatttca ctcaagattg ggtctcttta ccaggtgttc tacccgtggc ttcaggaggt 7440
attcacgttt ggcatatgcc tgctctgacc gagatctttg gggatgattc cgtactacag 7500
ttcggtggag gaactttagg acatccttgg atctgcagct agttctaga 7549

<210> 25
<211> 6465
<212> DNA
<213> Artificial Sequence

<220>
<223> Description of Artificial Sequence: Nucleotide sequence of DNA
      fragment from the vector pVTPA-Bar between the rice atpB and
      tobacco rbcL borders.

<400> 25
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240

accgcttgga tcgaagttat agatcccttt ttagtagaag taattctttc ttgcaaagaa 300
cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc ggggaaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggc ctttttggcc aagcttgata tcgaattccc ccgggctgct 1920
cccccgccgt cgttcaatga gaatggataa gaggctcgtg ggattgacgt gagggggcag 1980
ggatggctat atttctggga gcgaactccg ggcgaatacg aagcgcttgg atacagttgt 2040
agggagggat ttcatcgttt aaactcgagg tcgacggtat cgataagctt gatatcggta 2100
aggaggaata aacaatgagc ccagaacgac gcccggccga catccgccgt gccaccgagg 2160
cggacatgcc ggcggtctgc accatcgtca accactacat cgagacaagc acggtcaact 2220
tccgtaccga gccgcaggaa ccgcaggagt ggacggacga cctcgtccgt ctgcgggagc 2280
gctatccctg gctcgtcgcc gaggtggacg gcgaggtcgc cggcatcgcc tacgcgggcc 2340
cctggaaggc acgcaacgcc tacgactgga cggccgagtc gaccgtgtac gtctcccccc 2400
gccaccagcg gacgggactg ggctccacgc tctacaccca cctgctgaag tccctggagg 2460
cacagggctt caagagcgtg gtcgctgtca tcgggctgcc caacgacccg agcgtgcgca 2520
tgcacgaggc gctcggatat gccccccgcg gcatgctgcg ggcggccggc ttcaagcacg 2580
ggaactggca tgacgtgggt ttctggcagc tggacttcag cctgccggta ccgccccgtc 2640
cggtcctgcc cgtcaccgag atctgatgac ccgggggatc ctgatatcaa gcttctcgag 2700
tgaacgcgta tagggcccaa ttcgagctcg gtaccagcac caccagcggt gaggtgcgga 2760
acttctacaa cctcaaagcc cataacgttg cggatagaac ccttctcagg gtcaatcaga 2820
gcagcgtagt ttgctgcgtt cggcatcagt gctgccagaa tcgcagagta gctatctggg 2880
tcacagtaga acacacggtc agcagccgga acatagttct tggtcagagc cgcacgagcc 2940
ttagtcagag ccgcaataat ctccttaccc agcgcaactt ggtcggtcaa gtgcggcctt 3000
gttctgagtg gtctcaatta cggtagcagt acctaagccc tcgggggatc tggggaggaa 3060
tgagatatga aaaagcctga actcaccgcg acgtctgtcg agaagtttct gatcgaaaag 3120
ttcgacagcg tctccgacct gatgcagctc tcggagggcg aagaatctcg tgctttcagc 3180
ttcgatgtag gagggcgtgg atatgtcctg cgggtaaata gctgcgccga tggtttctac 3240
aaagatcgtt atgtttatcg gcactttgca tcggccgcgc tcccgattcc ggaagtgctt 3300
gacattgggg agtttagcga gagcctgacc tattgcatct cccgccgtgc acagggtgtc 3360
acgttgcaag acctgcctga aaccgaactg cccgctgttc tacaaccggt cgcggaggct 3420
atggatgcga tcgctgcggc cgatcttagc cagacgagcg ggttcggccc attcggaccg 3480
caaggaatcg gtcaatacac tacatggcgt gatttcatat gcgcgattgc tgatccccat 3540
gtgtatcact ggcaaactgt gatggacgac accgtcagtg cgtccgtcgc gcaggctctc 3600
gatgagctga tgctttgggc cgaggactgc cccgaagtcc ggcacctcgt gcacgcggat 3660
ttcggctcca acaatgtcct gacggacaat ggccgcataa cagcggtcat tgactggagc 3720
gaggcgatgt tcggggattc ccaatacgag gtcgccaaca tcttcttctg gaggccgtgg 3780
ttggcttgta tggagcagca gacgcgctac ttcgagcgga ggcatccgga gcttgcagga 3840
tcgccacgac tccgggcgta tatgctccgc attggtcttg accaactcta tcagagcttg 3900

gttgacggca atttcgatga tgcagcttgg gcgcagggtc gatgcgacgc aatcgtccga 3960
tccggagccg ggactgtcgg gcgtacacaa atcgcccgca gaagcgcggc cgtctggacc 4020
gatggctgtg tagaagtact cgccgatagt ggaaaccgac gccccagcac tcgtccgagg 4080
gcaaagaaat agagtaggta ccagcaccac cagcggtgag gtgcggaact tctacaacct 4140
caaagcccat aacgttgcgg atagaaccct tctcagggtc aatcagagca gcgtagtttg 4200
ctgcgttcgg catcagtgct gccagaatcg cagagtagct atctgggtca cagtagaaca 4260
cacggtcagc agccggaaca tagttcttgg tcagagccgc acgagcctta gtcagagccg 4320
caataatctc cttacccagc gcaacttggt cggtaagtgc ggccttgttc tgagtggtct 4380
caattacggt agcagtacct aagccctcgg gggatcagct tggctgtttt ggcggatgag 4440
agaagatttt cagcctgata cagattaaat cagaacgcag aagcggtctg ataaaacaga 4500
atttgcctgg cggcagtagc gcggtggtcc cacctgaccc catgccgaac tcagaagtga 4560
aacgccgtag cgccgatggt agtgtggggt ctccccatgc gagagtaggg aactgccagg 4620
catcaaataa aacgaaaggc tcagtcgaaa gactgggcct ttcgttttat ctgttgtttg 4680
tcggtgaacg ctctcctgag taggacaaat ccgccgggag cggatttgaa cgttgcgaag 4740
caacggcccg gagggtggcg ggcaggacgc ccgccataaa ctgccaggca tcaaattaag 4800
cagaaggcca tcctgacgga tggccttttt gcgtttctac aaactctttt tgtttatttt 4860
tctaaataca ttcaaatatg tatccgctgg gggatcagct tgatggccgc cagtgtgatg 4920
gatgggaagt tcttattatt taggttagtc aggtatttcc atttcaaaaa aaaaaaaagt 4980
aaaaaagaaa aattgggttg cgctatatat atgaaagagt atacaataat gatgtatttg 5040
gcaaatcaaa taccatggtc taataatcaa acattctgat tagttgataa ttcaaattgt 5100
gagcgctcac aatttgaaag attcctgtga aaagtttcat taacacggaa ttcgtgtcga 5160
gtagaccttg ttgttgtgag aattcttaat tcatgagttg tagggaggga tttatgtcac 5220
cacaaacaga gactaaagca agtgttggat tcaaagctgg tgttaaagag tacaaattga 5280
cttattatac tcctgagtac caaaccaagg atactgatat attggcagca ttccgagtaa 5340
ctcctcaacc tggagttcca cctgaagaag caggggccgc ggtagctgcc gaatcttcta 5400
ctggtacatg gacaactgta tggaccgatg gacttaccag ccttgatcgt tacaaagggc 5460
gatgctaccg catcgagcgt gttgttggag aaaaagatca atatattgct tatgtagctt 5520
accctttaga cctttttgaa gaaggttctg ttaccaacat gtttacttcc attgtaggta 5580
acgtatttgg gttcaaagcc ctgcgcgctc tacgtctgga agatctgcga atccctcctg 5640
cttatgttaa aactttccaa ggtccgcctc atgggatcca agttgaaaga gataaattga 5700
acaagtatgg tcgtcccctg ttgggatgta ctattaaacc taaattgggg ttatctgcta 5760
aaaactacgg tagagccgtt tatgaatgtc ttcgcggtgg acttgatttt actaaagatg 5820
atgagaacgt gaactcacaa ccatttatgc gttggagaga tcgtttctta ttttgtgccg 5880
aagcacttta taaagcacag gctgaaacag gtgaaatcaa agggcattac ttgaatgcta 5940
ctgcaggtac atgcgaagaa atgatcaaaa gagctgtatt tgctagagaa ttgggcgttc 6000
cgatcgtaat gcatgactac ttaacggggg gattcaccgc aaatactagc ttggctcatt 6060
attgccgaga taatggtcta cttcttcaca tccaccgtgc aatgcatgcg gttattgata 6120
gacagaagaa tcatggtatc cacttccggg tattagcaaa agcgttacgt atgtctggtg 6180
gagatcatat tcactctggt accgtagtag gtaaacttga aggtgaaaga gacataactt 6240
tgggctttgt tgatttactg cgtgatgatt ttgttgaaca agatcgaagt cgcggtattt 6300
atttcactca agattgggtc tctttaccag gtgttctacc cgtggcttca ggaggtattc 6360
acgtttggca tatgcctgct ctgaccgaga tctttgggga tgattccgta ctacagttcg 6420
gtggaggaac tttaggacat ccttggatct gcagctagtt ctaga 6465

<210> 26
<211> 7057
<212> DNA
<213> Artificial Sequence

<220>
<223> Description of Artificial Sequence: Nucleotide sequence of DNA
      fragment from the vector pVTPA-Estrep between the rice atpB and
      tobacco rbcL borders.

<400> 26
gtcgaggtca tgtcctgaag ttctttgtaa cgttgtaaag tttgcttaac tctttgtgca 60
gtttcataat gttcgttgcc aacgatccga ggttgtaaca tagttgaggt tgaatctaaa 120
ggatctactg caggataaat ccctttggaa gctaatcctc tggaaagtac ggtagtagca 180
tccaaatgtg caaatgttgt agcaggagca gggtcggtca aatcgtccgc aggtacataa 240
accgcttgga tcgaagttat agatcccttt ttagtagaag taattctttc ttgcaaagaa 300

cccatttctg tactaagagt aggttgataa cccactgcag agggcattct ccctaataag 360
gcagatacct ccgatcctgc ttgaacaaaa cgaaagatat tatcgatgaa tagaagcacg 420
tcttgcttat taacatctcg gaaatattct gccatagtta gggcagtcaa accaactctc 480
atacgagctc ctggcggttc attcatttgg ccatagacta gagctacctt tgattcctca 540
agattttttt cattaattac tccagattcc ttcatttcca tataaagatc atttccttca 600
cgagtccgtt cccctactcc gccaaatacg gatacgcccc cgtgagcttt agcaatattg 660
ttgattaatt ccatgatgag tactgtttta cctactccag ctcccccaaa tagtccgatt 720
tttcctccac gccgataagg agctaaaaga tcgaccacct taataccagt ttcaaagatg 780
gataatttcg tatctaactc gataaaggcg ggcgcggatc tatgaatagg gaatgttgca 840
ctagtatcta caggacccaa attgtcaaca ggctccccaa gaacgttgaa aattcgtcca 900
agagtagctc caccgacagg aacactgaga ggagctcccg tgtcaatcac ttccattcct 960
ctcatcaacc catctgtagc actcatagct acagctctaa ctcgattatt tcctaataat 1020
tgttgtacct cacaagttac attaatttgc ttaccgtcag tgtctcgact cttgactacc 1080
aaagcattat aaatataagg taacttgccc gggggaaaag tgacatccag cacgggtcca 1140
ataatttgat cgatacgccc tgtacttttt tcttcaattg tagaaacccc gggacgagaa 1200
gtagtaggat tggttctcat aattatcaca taattttcaa aaaaaaggaa tttatcgaaa 1260
ttttgatttt tttcttgttg aataatgcca aatcaacacc aaaaaaatat ccaaaaatcc 1320
aaaagtcaaa aggaaatgaa ttagttaatt caataagaga gaaaagggga ccagcacttg 1380
atttcgttgc ccaaacgaat cccattcaat cgtttactca tggaatgagt ccgtcggaaa 1440
gttcaatcaa tctttttttc atatacattt tgccttttgt aaacgatttg tgcctactct 1500
actttcttat ctaggacttc gatatacaaa atatatacta ctgtgaagca tagattgctg 1560
tcaacagaga attttcgtag tatttaggta tttccactca aaataagaaa agggggtcta 1620
ttaagaactt aataaggatt agaagttgat ttggggttgc gctatatcta ttaaagagta 1680
tacaataaag atggatttgg tgaatcaaat ccatggttta ataatcgaag catgttaact 1740
tacaataaca acaactcaag tcgaatgaat tcctatagca tagaatgtac acagggtgta 1800
cccattatat atgaatgaaa catattatat gaatgaaaca tattcattaa cttaagcatg 1860
ccccccattt tgtcgacggt atcgataagc ttgatatcga attcaccttg gttgacacga 1920
gtatataagt catgttatac tgttgaataa aaagccttcc attttgatta aataaaggag 1980
gattttcata tgtatcgatt acgtaaggag gaataaacca tgattgctgg acctgagtgg 2040
ctgctagacc gtccatctgt caacaacagc caattagttg ttagcgttgc tggtactgtt 2100
gaggggacga atcaagacat tagtcttaaa ttttttgaaa ttgacctaac atcacgacct 2160
gctcatggag gaaagacaga gcaaggctta agtccaaaat caaaaccatt tgctactgat 2220
agtggcgcga tgccacataa acttgaaaaa gctgacttac taaaggctat tcaagaacaa 2280
ttgatcgcta acgtccacag taacgacgac tactttgagg tcattgattt tgcaagcgat 2340
gcaaccatta ctgatcgaaa cggcaaggtc tactttgctg acaaagatgg ttcggtaacc 2400
ttgccgaccc aacctgtcca agaatttttg ctaagcggac atgtgcgcgt tagaccatat 2460
aaagaaaaac caatacaaaa tcaagcgaaa tctgttgatg tggaatatac tgtacagttt 2520
actcccttaa accctgatga cgatttcaga ccaggtctca aagatactaa gctattgaaa 2580
acactagcta tcggtgacac catcacatct caagaattac tagctcaagc acaaagcatt 2640
ttaaacaaaa cccacccagg ctatacgatt tatgaacgtg actcctcaat cgtcactcat 2700
gacaatgaca ttttccgtac gattttacca atggatcaag agtttactta ccatgtcaaa 2760
aatcgggaac aagcttatga gatcaataaa aaatctggtc tgaatgaaga aataaacaac 2820
actgacctga tctctgagaa atattacgtc cttaaaaaag gggaaaagcc gtatgatccc 2880
tttgatcgca gtcacttgaa actgttcacc atcaaatacg ttgatgtcaa caccaacgaa 2940
ttgctaaaaa gcgagcagct cttaacagct agcgaacgta acttagactt cagagattta 3000
tacgatcctc gtgataaggc taaactactc tacaacaatc tcgatgcttt tggtattatg 3060
gactatacct taactggaaa agtagaggat aatcacgatg acaccaaccg tatcataacc 3120
gtttatatgg gcaagcgacc cgaaggagag aatgctagct atcatttagc ctatgataaa 3180
gatcgttata ccgaagaaga acgagaagtt tacagctacc tgcgttatac agggacacct 3240
atacctgata accctaacga caaataagga tcctgatatc aagcttctcg agtgaacgcg 3300
tatagggccc aattcgagct cggtaccagc accaccagcg gtgaggtgcg gaacttctac 3360
aacctcaaag cccataacgt tgcggataga acccttctca gggtcaatca gagcagcgta 3420
gtttgctgcg ttcggcatca gtgctgccag aatcgcagag tagctatctg ggtcacagta 3480
gaacacacgg tcagcagccg gaacatagtt cttggtcaga gccgcacgag ccttagtcag 3540
agccgcaata atctccttac ccagcgcaac ttggtcggtc aagtgcggcc ttgttctgag 3600
tggtctcaat tacggtagca gtacctaagc cctcggggga tctggggagg aatgagatat 3660
gaaaaagcct gaactcaccg cgacgtctgt cgagaagttt ctgatcgaaa agttcgacag 3720
cgtctccgac ctgatgcagc tctcggaggg cgaagaatct cgtgctttca gcttcgatgt 3780
aggagggcgt ggatatgtcc tgcgggtaaa tagctgcgcc gatggtttct acaaagatcg 3840
ttatgtttat cggcactttg catcggccgc gctcccgatt ccggaagtgc ttgacattgg 3900
ggagtttagc gagagcctga cctattgcat ctcccgccgt gcacagggtg tcacgttgca 3960

agacctgcct gaaaccgaac tgcccgctgt tctacaaccg gtcgcggagg ctatggatgc 4020
gatcgctgcg gccgatctta gccagacgag cgggttcggc ccattcggac cgcaaggaat 4080
cggtcaatac actacatggc gtgatttcat atgcgcgatt gctgatcccc atgtgtatca 4140
ctggcaaact gtgatggacg acaccgtcag tgcgtccgtc gcgcaggctc tcgatgagct 4200
gatgctttgg gccgaggact gccccgaagt ccggcacctc gtgcacgcgg atttcggctc 4260
caacaatgtc ctgacggaca atggccgcat aacagcggtc attgactgga gcgaggcgat 4320
gttcggggat tcccaatacg aggtcgccaa catcttcttc tggaggccgt ggttggcttg 4380
tatggagcag cagacgcgct acttcgagcg gaggcatccg gagcttgcag gatcgccacg 4440
actccgggcg tatatgctcc gcattggtct tgaccaactc tatcagagct tggttgacgg 4500
caatttcgat gatgcagctt gggcgcaggg tcgatgcgac gcaatcgtcc gatccggagc 4560
cgggactgtc gggcgtacac aaatcgcccg cagaagcgcg gccgtctgga ccgatggctg 4620
tgtagaagta ctcgccgata gtggaaaccg acgccccagc actcgtccga gggcaaagaa 4680
atagagtagg taccagcacc accagcggtg aggtgcggaa cttctacaac ctcaaagccc 4740
ataacgttgc ggatagaacc cttctcaggg tcaatcagag cagcgtagtt tgctgcgttc 4800
ggcatcagtg ctgccagaat cgcagagtag ctatctgggt cacagtagaa cacacggtca 4860
gcagccggaa catagttctt ggtcagagcc gcacgagcct tagtcagagc cgcaataatc 4920
tccttaccca gcgcaacttg gtcggtaagt gcggccttgt tctgagtggt ctcaattacg 4980
gtagcagtac ctaagccctc gggggatcag cttggctgtt ttggcggatg agagaagatt 5040
ttcagcctga tacagattaa atcagaacgc agaagcggtc tgataaaaca gaatttgcct 5100
ggcggcagta gcgcggtggt cccacctgac cccatgccga actcagaagt gaaacgccgt 5160
agcgccgatg gtagtgtggg gtctccccat gcgagagtag ggaactgcca ggcatcaaat 5220
aaaacgaaag gctcagtcga aagactgggc ctttcgtttt atctgttgtt tgtcggtgaa 5280
cgctctcctg agtaggacaa atccgccggg agcggatttg aacgttgcga agcaacggcc 5340
cggagggtgg cgggcaggac gcccgccata aactgccagg catcaaatta agcagaaggc 5400
catcctgacg gatggccttt ttgcgtttct acaaactctt tttgtttatt tttctaaata 5460
cattcaaata tgtatccgct gggggatcag cttgatggcc gccagtgtga tggatgggaa 5520
gttcttatta tttaggttag tcaggtattt ccatttcaaa aaaaaaaaaa gtaaaaaaga 5580
aaaattgggt tgcgctatat atatgaaaga gtatacaata atgatgtatt tggcaaatca 5640
aataccatgg tctaataatc aaacattctg attagttgat aattcaaatt gtgagcgctc 5700
acaatttgaa agattcctgt gaaaagtttc attaacacgg aattcgtgtc gagtagacct 5760
tgttgttgtg agaattctta attcatgagt tgtagggagg gatttatgtc accacaaaca 5820
gagactaaag caagtgttgg attcaaagct ggtgttaaag agtacaaatt gacttattat 5880
actcctgagt accaaaccaa ggatactgat atattggcag cattccgagt aactcctcaa 5940
cctggagttc cacctgaaga agcaggggcc gcggtagctg ccgaatcttc tactggtaca 6000
tggacaactg tatggaccga tggacttacc agccttgatc gttacaaagg gcgatgctac 6060
cgcatcgagc gtgttgttgg agaaaaagat caatatattg cttatgtagc ttacccttta 6120
gacctttttg aagaaggttc tgttaccaac atgtttactt ccattgtagg taacgtattt 6180
gggttcaaag ccctgcgcgc tctacgtctg gaagatctgc gaatccctcc tgcttatgtt 6240
aaaactttcc aaggtccgcc tcatgggatc caagttgaaa gagataaatt gaacaagtat 6300
ggtcgtcccc tgttgggatg tactattaaa cctaaattgg ggttatctgc taaaaactac 6360
ggtagagccg tttatgaatg tcttcgcggt ggacttgatt ttactaaaga tgatgagaac 6420
gtgaactcac aaccatttat gcgttggaga gatcgtttct tattttgtgc cgaagcactt 6480
tataaagcac aggctgaaac aggtgaaatc aaagggcatt acttgaatgc tactgcaggt 6540
acatgcgaag aaatgatcaa aagagctgta tttgctagag aattgggcgt tccgatcgta 6600
atgcatgact acttaacggg gggattcacc gcaaatacta gcttggctca ttattgccga 6660
gataatggtc tacttcttca catccaccgt gcaatgcatg cggttattga tagacagaag 6720
aatcatggta tccacttccg ggtattagca aaagcgttac gtatgtctgg tggagatcat 6780
attcactctg gtaccgtagt aggtaaactt gaaggtgaaa gagacataac tttgggcttt 6840
gttgatttac tgcgtgatga ttttgttgaa caagatcgaa gtcgcggtat ttatttcact 6900
caagattggg tctctttacc aggtgttcta cccgtggctt caggaggtat tcacgtttgg 6960
catatgcctg ctctgaccga gatctttggg gatgattccg tactacagtt cggtggagga 7020
actttaggac atccttggat ctgcagctag ttctaga 7057

## Description

### Field of the Invention.

This invention is related to the field of Biotechnology, and more specifically to the Genetic Engineering of Plants. In particular, here are provided DNA constructs useful for the efficient introduction and expression of foreign genes in Angiosperm plant plastids. The usefulness of these genetic constructions to obtain with high efficiency transplastomic plants that express heterologous proteins, particularly vaccine antigens and proteins of pharmaceutical use is demonstrated.

### Background of the Invention.

The genetic engineering of plants is a technology that has shown to be very productive for basic research and commercial production of new biotechnological products (Hammond J. Curr. Top. Microbiol. Immunol 1999, **240**:1-19; Simoens C. and Van Montagu M. Reproduction Update 1995, **1**:523-542).

Until some years ago, the production of transgenic plants by direct or Agrobacterium-mediated transformation methods, was focused to the stable insertion of the chimeric genes into the nucleus of the plant cell (see: Hansen G. and Chilton M.D. Curr. Top. Microbiol. Immunol. 1999, **240**:21-57; Newell C.A. Mol. Biotechnol 2000, **16**:53-65). However, some limitation that present the nuclear transformation can be only overcome by the selective introduction of the new genes into the plant cell organelles (see: Bogorad L. Trends in Biotechnology 2000, **18**:257-263; Heifetz P.B. Biochimie 2000, **82**:655-666). In particular, the expression of heterologous genes in plastids presents the following advantages with regard to nuclear expression: 1) the expression level of the transgene is 10 to 50 times higher than in the nucleus due to high copy number of plastid genomes per cell; 2) the insertion of the new genes by homologous recombination is site-specific, avoiding positional effects, gene silencing and others undesirable phenomena that affect expression of genes introduced into the plant nucleus; 3) policistronic gene expression is possible, which is very desirable when trying to make engineering of metabolic pathways or altering multigenic characters; 4) the plastid genetic information is mainly maternally inherited, reducing the risk of possible damages to Nature due to the spreading of chimeric genes in the environment through pollen. Additionally, gene expression in plastids offers the possibility of accumulating heterologous proteins in a new microenvironment that could provide a greater stability and/or an easier purification process.

Different methods have been reported for the introduction of genes into plastids of algae and higher plants. In particular, it has been frequently used the bombardment with accelerated microparticles covered with DNA (Boynton J.E; Gilham E.H; Hosler J.P; and others. Science 1988, **240**:1534-1538; Daniell H; Vivekananda J; Nielsen B; and others. Proc. Natl. Acad. Sci. USA 1990, **87**:88-92; Svab Z; Hajdukiewicz P; Maliga P. Proc. Natl. Acad. Sci. USA 1990, **87**:8526-8530), and in less proportion the transformation of plant protoplasts by PEG (O'Neill C; Horvath G.V; Horvath E; and others. Plant J 1993, **3**:729-738; Golds T; Maliga P; Koop H-U. Biotechnology 1993, **11**:95-97), and the microinjection (Knoblauch M; Hibberd J.M; Gray J.C; van Bel A.J.E. Nature Biotechnol 1999,17:906-909).

The stable insertion of the introduced DNA sequences into plastid genome has been achieved often by using the efficient mechanisms of homologous recombination that naturally occur in these cellular organelles (Blowers A.D; Bogorad L; Shark K.B.; Sanford J.C. Plant Cell 1989, **1**:123-132; Staub J.M. and Maliga P. Plant Cell 1992, **4**:39-45; Kavanagh T.A; Thanh N.D; Lao N.T; and others. Genetics 1999, **152:**1111-1122). It is important to emphasize that the larger the DNA regions with homology the higher the frequency of the recombination events, being these negligible when the homologous regions are smaller than 137 bp (Zoubenko O.V; Allison L.A; Svab Z; Maliga P. Nucleic Acid Res 1994, **22**:3819-3824).

Many regions have been proposed as suitable recombination sites for inserting chimeric sequences in plant plastids: rbcL-accD, psbE-petA, trnV-16SrRNA operon, psbA-trnH, trnA-trnI, 23SrRNA region, etc (Svab Z. and Maliga P. Proc. Natl. Acad. Sci. USA 1993, **90**:913-917; Zoubenko O.V; Allison L.A; Svab Z; Maliga P. Nucleic Acid Res 1994, **22**:3819-3824; Daniell H; Datta R; Varma S; and others. Nature Biotechnol 1998, **16**:345-348; US Pat. 5,877,402; WO 9910513). However, in spite of some of these regions have been suggested as "universals" for the integration of foreign DNA sequences in plastid genomes of different plant species, their universality presents limitations due to one or many of the following reasons: 1) their sequences and/or structures are not highly conserved among the plastid genomes of different plant species; 2) they posses very short regions of high homology followed, or interrupted by, introns or others regions much less conserved (which decreases the homologous recombination frequency and therefore the obtaining of plants with transgenic plastids (transplastomics)); 3) they are located in some cases forming part of plastid operons, and it is known that an alteration inside of these structures can affect the wild transcription level for each operon's gene or the correct processing of policistronic RNA (Ohme M; Kamogashira T; Shinozaki K; Sugiura M. Nucleic Acid Res 1985, 13:1045-1056), with the undesirable consequences this causes to plastid metabolism. In fact, it is not fortuitous that using vectors containing the regions above mentioned as recombination sites, only there have been reported in peer-reviewed publications the production of transplastomic plants from tobacco (Svab Z. and Maliga P. Proc. Natl. Acad. Sci. USA 1993, **90**:913-917), potato (Sidorov V.A; Kasten D; Pang S.Z; and others. Plant J 1999, **19**:209-216) and tomato (Ruf S; Hermann M; Berger I.J; and others. Nature Biotechnol 2001, **19**:870-875), all them Solanaceus species very close evolutionarily and at level of the DNA primary structure. In addition, vectors that use these recombination regions show a low frequency of production of homoplasmic transplastomic plants (plants with all their plastid genomes homogeneously transformed), probably because these vectors include the use of terminator regions and promoters of plastid origin larger than 174 bp (Iamtham S. and Day A. Nature Biotechnol 2000, **18**:1172-1176), which causes that many transformation events fail due to recombination between these vector elements and the homologous regions of the plastid genome, giving rise to non viable plastids (Svab Z. and Maliga P. Proc. Natl. Acad. Sci. USA 1993, **90**:913-917; Kanevski I; Maliga P; Rhoades D.F; Gutteridge S. Plant Physiol 1999, **119**:133-141).

The region of the plastid genome more universally conserved by structure and nucleotide sequence between Angiosperm plants is that composed by the divergently transcribed rbcL and atpB operons. In fact, these genes contain DNA segments larger than 1500 bp with homologies superior to 85% between plastids of plants belonging to different classes (dicotyledonous or monocotyledonous) and more than 91% of nucleotide homology when analyzing plants of the same class (see Table 1). On the other hand, the intergenic region between these operons presents a very low nucleotide homology (less than 50%) among the plastids genomes of dicotyledonous and monocotyledonous plants.

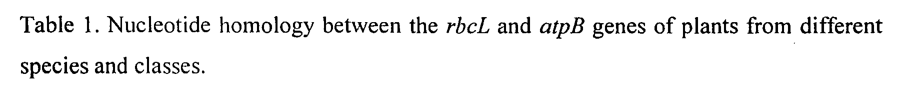

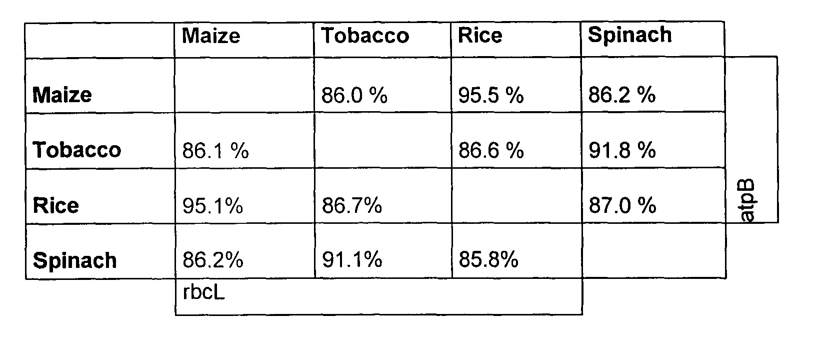

The large subunit of the ribulose-1,5-bisfosfate carboxilase-oxigenase (Rubisco) is codified by the plastid gene *rbcL.* It has been determined experimentally that the carboxyl-terminal region of rbcL subunit forms the alpha-helix number 8 of this protein, and that this region is involved in the interaction with the small subunit of this enzyme, codified by the nuclear gene *rbcS* (Knight S; Andersson I; Branden C-I. J. Mol. Biol. 1990, **215**:113-160; Curmi P.M.G; Cascio D; Sweet R.M; and others. J. Biol. Chem. 1992, **267**:16980-16989). Starting from these and others data (Kanevski I; Maliga P; Rhoades D.F; Gutteridge S. Plant Physiol. 1999, **119**:133-141), and considering the small degree of conservation between C-terminal regions of plastid rbcL proteins belonging to different plant species, it was put forward the hypothesis that the alpha-helix 8 of rbcL defines the species-specificity of the interactions between both subunits of Rubisco enzyme.

It has been shown that possibly due to a loop-like structure that is located near to the transcription start of *rbcL* promoter (position +1 to +26 starting from the beginning of transcription) the produced messenger RNA become stable, that way compensating the decrease in transcription activity of this promoter during the darkness (Shiina T; Allison L; Maliga P. The Plant Cell 1998, **10**:1713-1722). Likewise, various segments of sequences sited near or inside the structural region of this promoter resemble sequences of chloroplast DNA binding factors (CDF), and could play an important role in transcriptional regulation of the rbcL promoter.

Some features that characterize the transcription/translation of atpB-atpE operon (which encodes for β and ε subunits of the chloroplast ATP-synthase) have been reported (Shinozaki K; Deno H; Kato A; Sugiura M. Gene 1983, **24**:147-155; Gatenby A.A; Rothstein S.; Nomura M. Proc. Natl. Acad. Sci. USA 1989, **86**:4066-4070; Kapoor S; Wakasugi T; Deno H; Sugiura M. Curr. Genet. 1994, **26**:263-268). These two genes are overlapped and in particular, the translational features of *atpE* call the attention because they are based on a frame-shift of the open reading frame, and on a conserved region inside the atpB coding sequence which contains a possible promoter for atpE (positions +1027 to +1064 of the nucleotide sequence that encodes for atpB protein of rice, tobacco, etc.). The functionality of this promoter "in vivo" has not been proved, but it is not discarded that it could play some role in transcriptional regulation of atpE expression.

The possible use atpB-rbcL intergenic region, as target for the insertion of foreign genes in plastids, was only described by Cannon M.C. and Cannon F.C in the European Application Patent No.0251654. However, this patent proposed the use of a bacterial transposon as carrier molecule for introducing foreign genes into the intergenic region between these two divergently transcribed operons. It is not casual that Cannon M.C. and

Cannon F.C. did not succeeded in obtaining transplastomic plants, because the insertion of a transposon in this intergenic region not only interrupts the reported interaction between atpB and rbcL promoters (Hanley-Bowdoin L. and Chua N-H. Mol. Gen. Genet. 1989, **215**:217-224), but also (as it is for maize and rice, for example) it interrupts the transcription of the *atpB* gene starting from the NEP promoter (nuclear polymerase dependent promoter) that is located inside the 5' untranslated region of the *rbcL* gene (Welhe A. and Börner T. Trends in Plant Science 1999, **4**:169-170). It is evident that an accurate transcription and regulation of expression of the products codified by these operons are absolutely necessary for the correct functionality and viability of plastids. Additionally, the expression vector described by Cannon M.C. and Cannon F.C. does not contain transcription terminators, an indispensable element for proper gene expression in plastids (Stern D.B. and Kindle K.L. Mol. Cell Biol. 1993, **13**:2277-2285; Chen H.C. and Stern D.B. Mol Cell Biol. 1991, **11**:4380-4388).

In addition to transcription terminator regions, which can be of bacterial origin (Chen L.J. and Orozco E.M. Jr. Nucleic Acid Res. 1988, **16**:8411-8431), it is necessary to put the genes introduced into plastids under the control of functional promoters in this organelle. Among the most used promoters for expressing genes in transgenic plastids are the strong promoters Prrn of 16S rRNA (Svab Z. y Maliga P. Proc. Natl. Acad. Sci. USA 1993, **90**:913-917) and PsbA (Daniell H. Methods in Molecular Biology 1997, **62**:463-489). The Prrn promoter is constitutive, while the transcription/translation of RNA produced by the PsbA promoter is induced by light and depends on its 5' untranslated region. Deleting the 5' untranslated region of the *psbA* gene makes its mRNA expression light independent, although this affects the translation (Staub J.M. y Maliga P. Plant J. 1994, **6**:547-553; Eibi C; Zou Z; Beck A; and others. Plant J. 1999, **19**:333-345). These promoters have -35 and -10 consensus sequences homologous to those described for bacterial promoters and their transcription is dependent of a polymerase codified by the plastid genome (PEP) (Hess W. and Börner T. Int. Rev. Cytol. 1999, **190**:1-59).

Among others similarities between transcriptional/translational mechanisms of plastids and bacteria, it has also been proved that policistronic mRNA can be expressed and efficiently translated in tobacco plastids (Staub J.M. and Maliga P. Plant J. 1995, 7:845-848); being functional in chloroplasts the ribosome binding sites (RBS) that comprise Shine-Dalgarno like sequences (Stern D.B; Higgs D.C; Yang J. Trends Plant Sci. 1997, **2**:308-315).

To facilitate the selection of transplastomic plants, it has been proposed among others alternatives the use of positive selection markers as for example: kanamycin (Daniell H. Methods in Molecular Biology 1997, **62**:463-489), or the most universally used spectinomycin (Svab Z; Hajdukiewicz P; Maliga P. Proc. Natl. Acad. Sci. USA 1990, **87**:8526-8530; Svab Z. and Maliga P. Proc. Natl. Acad. Sci. USA 1993, **90**:913-917); for this purpose the gene that confers resistance to the selection agent is cloned in the transformation vector under appropriate controlling signals for its correct expression in plastids.

To protect the Nature, there have been designed "clean" technologies that allow the removal of genes that encode for selection markers from plants that are released to the environment. Two of these technologies successfully applied to the production of transplastomic plants are the following: co-transformation with two vectors and the subsequent segregation and elimination of individuals that carry the selection marker (Fisher N; Stampacchia O; Redding K; Rochaix J.D. Mol. Gen. Genet. 1996, **251**:373-380), and the insertion of marker genes between two directly repeated sequences in the expression cassette to allow the removal of these genes by homologous recombination during subsequent culture cycles in non selective medium (Fisher N; Stampacchia O; Redding K; Rochaix J.D. Mol. Gen. Genet. 1996, **251**:373-380; lamtham S. and Day A. Nature Biotechnol. 2000, **18**:1172-1176).

### Detailed description of the invention.

The vector for stable transformation and expression of heterologous genes in plastids of Angiosperm plants that proposes the present invention comprises some own features that distinguish it: 1) it is not use a transposon to insert DNA fragments into plastid genomes; 2) the atpB and rbcL border regions belong to Angiosperm plants of different classes, forming an artificial intergenic region; 3) multiple cloning sites in the artificial intergenic region transcriptionally inactive, allow the insertion of one or several heterologous genes without affecting the correct transcription, mRNA processing and the expression of the *atpB* and *rbcL* genes; 4) the design of this vector allows the expression of introduced genes without the necessity of carrying promoter and terminator regions, whenever the sequences that encodes for protein are preceded by a ribosome binding site ( this contribute to reduce undesirable recombination events and to save plastid metabolic resources, allowing a greater stability of the introduced heterologous DNA fragments and an increase in the production frequency of homoplasmic plants); 5) the structure and sequence of border regions proposed by the present invention, guarantee the vector universality. Additionally, the vector herein provided constitutes a "clean" tool for introducing genes into crop plants due to a design that allows the removal of selection marker, by homologous recombination between directly repeated sequences, once the homoplasmic transplastomic plants have been obtained.

The design and extension of the atpB-rbcL region, with its artificial intergenic region, constitute the base of the present invention object, since it allows us to insert genes and operons in an efficient and stable way into plastid genomes of Angiosperm plants of any species, without causing a functional alteration.

For designing the artificial intergenic region it was necessary to resolve the challenge that represents the introduction of a foreign gene in this region, without affecting the interaction between rbcL and atpB promoters, and their wild transcriptional levels.

On the other hand, it was also necessary to consider that the *atpB* and *rbcL* gene products constitute subunits of enzymes that carry out vital functions for the maintenance of plastids and plants. This situation imposes limitations to the length of fragments selected as borders for recombination, because it is advisable that certain domains of these proteins will be involved in protein-protein interactions and could determine the specificity of recognition between different subunits of these enzymes and their correct folding and functioning.

To use the atpB-rbcL region as a recombination site for foreign genes insertion in plastids of different plant species, it is important to consider that hybrid proteins will be produced, and therefore what it was mentioned above deserves an especial attention. In this way, to avoid interferences with the correct folding and functioning of hybrid Rubisco, it was decided to maintain invariable during the recombination event the *rbcL* gene region that encodes for the alpha helix 8. Thus, during the design of the vector aim of this invention it was decided that the DNA fragment for plastid transformation by homologous recombination, containing part of the translatable *rbcL* gene sequence (further called **rbcL-border**), should extend only till N-terminal region of alpha helix 8, and more specifically till a tryptophan (Trp-411) highly conserved in this position among rbcL proteins from Angiosperm plants.

Concerning the region that encodes for the *atpB* gene, it is not detected any domain with a marked variability when comparing protein sequences from plastids of different plant species. However, the transcription/translation features of atpB-atpE operon were taken into consideration to select the DNA fragment, containing part of the translatable *atpB* gene sequence, (further called **atpB-border**) for the integration of foreign genes into plastids by homologous recombination.

To design the artificial intergenic region that allows the insertion of new genes into plastids without affecting the atpB and rbcL transcription rates, we took advantage of the low homology at nucleotide level that exhibit the region between these operons when comparing plastids sequences belonging to Angiosperm plants of different species.

In the genetic construction aim of this invention, the **rbcL-border** should comprise a DNA fragment that bears part of the gene and the whole 5' regulatory region. This fragment includes: 1) the *rbcL* gene PEP promoter (dependent on plastid encoded polymerase) with its associated CDF sequences; 2) the 5' untranslated region of the *rbcL* gene and 3) the sequence that encodes for the rbcL protein till the region that corresponds to the N-terminal of alpha helix 8. Thus, the **rbcL-borde**r in the vector aim of this invention comprises a DNA fragment which extends from the position -291 of nucleotide sequence that encodes for this protein in **tobacco**, to position +1233 (Trp-411 in the aminoacid sequence) starting from the translation start codon of this gene.

It is necessary to point out when attempting to clone the rbcL-border, we discovered that expression of the *rbcL* gene starting from its own promoter is toxic to E. *coli.* Therefore, it was necessary to replace a little conserved fragment from the 5' untranslated region of this gene for an "ideal" lac operator (Simons A; Tils D; von Wilcken-Bergmann B; Muller-Hill B. Proc. Natl. Acad. Sci. USA 1984, **81**:1624-1628) and also, to clone this chimeric border into a low copy number plasmid vector (pBR322) that will be propagated in a *lacl*^{*q*} *E. coli* strain, as for example XL-1 blue, JM 101, etc.

In the genetic construction aim of this invention it was decided to take as a**tpB-border** a DNA fragment that includes: 1) the adjacent rbcL promoter and the stem-loop forming sequences near to the transcription start point that stabilize the mRNA produced by this promoter; 2) the promoters of the *atpB* gene including its NEP promoter; 3) the 5' untranslated region of the *atpB* gene and 4) the sequence that encodes for atpB till 300 bp before the stop codon of the gene. In this way, when designing the vector aim of this invention the **atpB-border** extends from position -654 of nucleotide sequence that encodes for this protein in rice, to position +1211 starting from the translation start codon of this gene.

In the atpB-border, the rbcL promoter adjacent to *atpB* gene allows the stable transcription and expression of genes of interest in opposite direction to the transcription of the atpB-atpE operon. At user's convenience, under this rbcL promoter could be possible to clone a genetic cassette containing one or more foreign genes controlled by a promoter functional in plastids, so the expression of these genes will be enhanced by the transcription from two promoters in tandem.

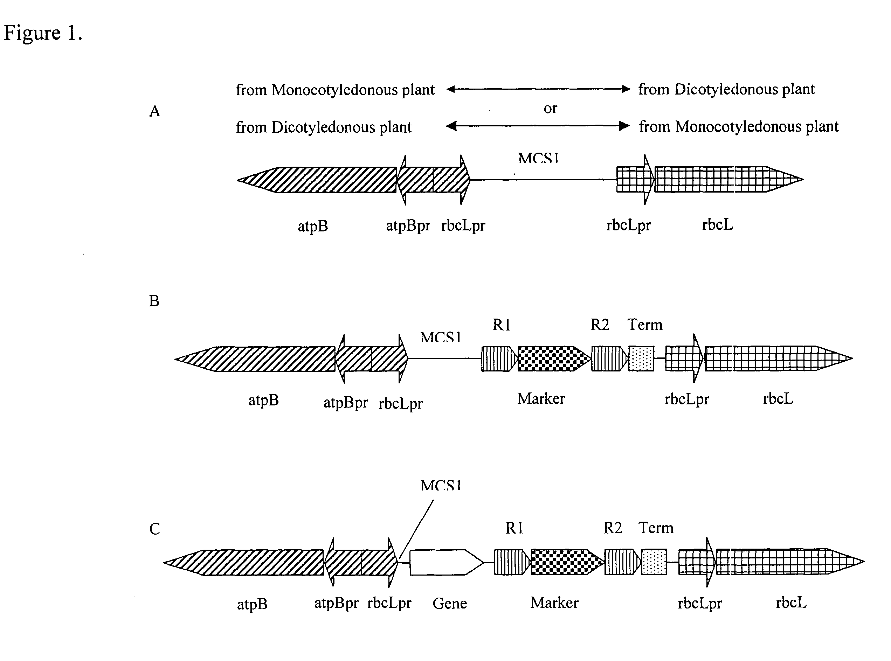

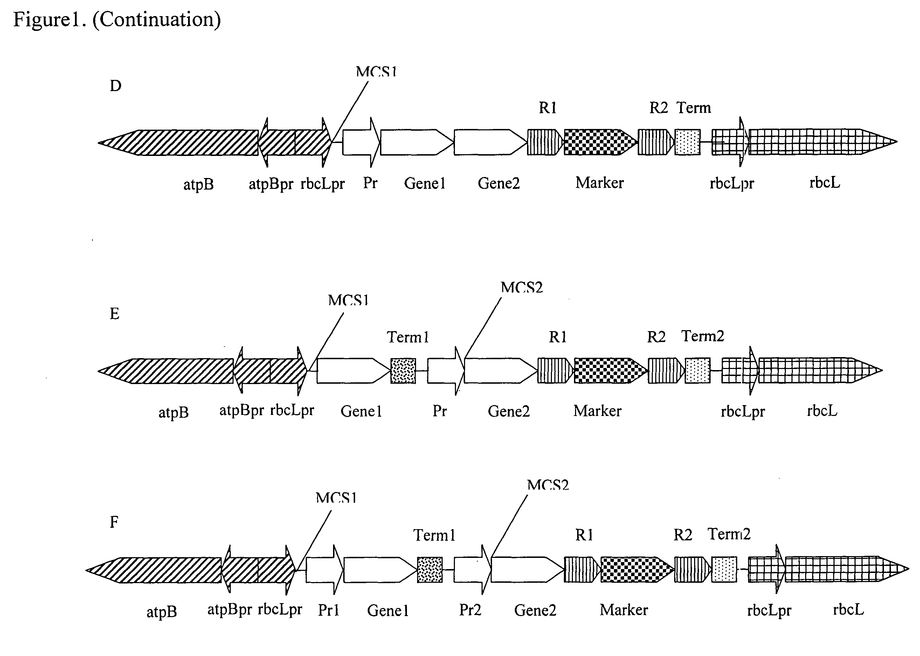

The borders regions previously described are introduced into a plasmid vector, for their maintenance and multiplication in *E. coli,* separated by a DNA segment that contains multiple cloning sites (MCS 1, see Figure 1-A), useful for insertion of heterologous genes that further will be introduced into plastid genomes. According to this structure, in which the *atpB* and *rbcL* genes will be divergently transcribed and due to rbcL promoter region is duplicated, two directly repeated DNA fragments are obtained bordering the region that contains the cloning sites. However, owing to the low homology at nucleotide level in this intergenic region between plastid genomes from different Angiosperm plant species, it is not foreseen any recombination event in our construction because only a small fragment of 90 bp has a high degree of homology.

The construction previously described is the base of the vector aim of this invention, where a gene encoding for a selectable marker that allows the positive selection of transformed cells will be introduced into the MCS1 ( in direction to the rbcL-border, starting from the rbcL promoter of the atpB-border), bordered by directly repeated DNA sequences that will enable the removing of the marker gene by homologous recombination, once homoplasmic transplastomic plants have been obtained. The transcription termination of the marker gene, or other gene introduced into the MCS1, will be carried out by a bacterial terminator located adjacent to the 5' end of the rbcL-border (preferably the rrnBT1T2 bi-directional terminator of rrnB operon of E.coli (Brosius J; Dull T.J; Sleeter D.D; Noller H.F. J. Mol. Biol. 1981, **148**:107-127), thus avoiding that transcription of inserted genes interfere the normal functioning of the rbcL-border promoter.

In this way, the general structure of the vector aim of this invention is shown in Figure 1-B, where the gene o genes of interest are inserted under the rbcL promoter of the atpB-border forming a transcriptional unit mono or polycistronic (Figure 1-C), or under other plastid functional promoters allowing the obtainment of, for example, a polycistronic unit under two promoters in tandem (Figure 1-D), or two independent transcriptional units under different combinations of promoter regions (Figure 1-E and Figure 1-F).

For facilitating the selection of transplastomic plants, it was decided to use the hygromycin as selection marker; although this antibiotic is extremely toxic to dicotyledonous plants as tobacco, and therefore it should be used carefully, it results more effective as selection agent for the obtainment of monocotyledonous plants with genetically modified plastids. In this way, the *hgh* gene that encodes for resistance to hygromycin B in *Klebsiella* (Gritz L. and Davies J. Gene 1983, **25**:179-188), containing an appropriate Shine-Dalgarno region, is cloned into the MCS1 under rbcL promoter of the atpB-border (Figure 1-B).

With the purpose of offering an ecological tool, in the vector aim of this invention the marker gene was introduced bordered by two directly repeated DNA sequences that allow its removing by homologous recombination (Figure 1-B), once the selective agent is eliminated from the culture media when the homoplasmic transplastomic plants have been already obtained. The repeated sequences used in this construction came from a DNA region that encodes for the s 10 protein of phage T7 (Dunn J.J. and Studier F.W. J. Mol. Biol. 1983, **166**:477-535). These vectors have the peculiarity of removing only the marker gene, so that the rest of operon regulatory sequences are maintained invariable (Figures 1-C, 1-D, 1-E and 1-F); therefore, any gene transcriptionally fused to marker gene will unchangeable continue its expression, in this way economizing the vector resources. This architecture is another of the qualities that distinguish the vector aim of this invention from others described systems as vehicles for genetic transformation of plastids.

It is obvious that others genes which confer resistance to antibiotics as to spectinomycin (Chinault A.C; Blaskeley V.A; Roessler E; Willis D.G; and others. Plasmid 1986, **15**:119-131), or to sulphonamides (Guerineau F. and Mullineaux P. Nucleic Acid Res 1989, **17**:4370), could be also used to allow the selection of transplastomic plants. Likewise, genes that confer resistance to herbicides like glyfosate (Shah D.M; Horsch R.B; Klee H.J; Kishore G.M; and others. Science 1986, **233**:478-481; Comai L; Facciotti D; Hiatt W.R; Thompson G; and others. Nature 317:741-744), gluphosinate (DeBlock M; Botterman J; Vandewiele M; Dockx J; and others. EMBO Journal 1987, **6**:2513-2518), asulam (Guerineau F; Brooks L; Meadows J; Lucy A; and others. Plant Mol Biol 1990, **15**:127-136), bromoxinil (Stalker D.M. and McBride K.E. J. Bacteriol 1987, **169**:955-960), sulphonilureas and imidazolinones (LaRossa R.A and Smulky D.R. J. Bacteriol 1984, **160**:391-394), etc, can be cloned in the artificial intergenic region and used as selection markers with the advantage that herbicide tolerance is an agronomical desirable character.

For somebody skilled in the state of the art and genetic engineering techniques, it is obvious that what is described here allows the use of any combination of atpB and rbcL regions, with all or parts of the described features, as border regions for integration of genes into plastid genomes whenever these borders come from plastids of Angiosperm plants of different classes (monocotyledonous or dicotyledonous). Likewise, multiple genes, selection markers and combinations of cistrons and operons can be cloned into the MCS1 of the artificial intergenic region of the vector aim of this invention (Figure 1-A), in order to stably insert and express them in plastids of any Angiosperm plant.

### Deposit of microorganisms.

The plasmid vectors pVTPA-HB-aadA, pVTPA-f-GUS and pVIEP were deposited under the Budapest Treaty Rules in the *Belgian Coordinated collection of Microorganisms BCCM, LMBP-COLLECTION* with the numbers LMBP 4635, LMBP 4636 and LMBP 4637 respectively deposited on September 24, 2002.

### Description of the drawings.

Figure 1. A, basic structure for the construction of the vector aim of the present invention showing the region that contains the multicloning sites (MCS1) limited b 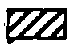 atpB bor 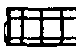 and rbcL border belonging to Angiosperm plants of different classes (monocotyledonous or dicotyledonous).
   B, general structure of the vector aim of the present invention (rbcLpr-promoter of the *rbcL* gene, atpBpr-promoter of the *atpB* gene, Term-transcription terminator of non-plastid origin, marker-selection marker gene, R1, R2-direct repeated sequences that allow the removing of marker gene by homologous recombination).
   C, D, E, F, variants of the vector aim of the present invention showing the possibilities of expressing one or more genes of interest (gene, gene1, gene2) as monocistronic units (E, F), bicistronics (C, E, F) or tricistronics (D), under the control of one (C, E, F) or more (D, F) plastid functional promoters (Pr, Pr1, Pr2).
Figure 2. Map of the vector for transformation of plastids of Angiosperm plants, pVTPA-f.
Figure 3. Map of the vector for transformation of plastids of Angiosperm plants, pVTPA.
Figure 4. Map of the intermediary vector for the expression of genes in plastids, pVIEP (S/D-Shine-Dalgarno sequence).
Figure 5. Southern blot of 10 µg of chloroplast DNA purified by sucrose gradient, digested with *Pst*I and using as probe the 5' fragment of the *rbcL* gene labeled with ³²P. Lane 1: non-transformed plant; lanes 2-6: transformed plants (it can be appreciated that all plants are homoplasmic); lanes 7 and 8: transformed plants grown in absence of selection agent during the last cycle of *in vitro* tissue culture (it can be noted in lane 7 that the marker gene has not been removed totally).
Figure 6. Western blot for demonstrating the use of the transformation vectors aim of this invention for expressing the Hepatitis B surface antigen in chloroplasts: 10 µg of total leaves proteins were applied in each lane. Lane 1: non-transformed plant; lanes 2-5: transplastomic plants expressing the HbsAg (it can be observed the signal revealed by the antibody against the Hepatitis B virus at the expected molecular weight of 25 kDa).
Figure 7. Western blot for demonstrating the use of the transformation vectors aim of this invention for expressing in plastids the heavy and light chains that conform a monoclonal antibody: 10 µg of total leaves proteins were applied in each lane. Lane 1: non-transformed plant; lanes 2-5: transplastomic plants expressing the recombinant antibody CB-Hep.1 (it can be observed the signals corresponding to heavy (50 kDa) and light (25 kDa) chains of the inmunoglobulin).

### Realization Examples.

Example No.1. Construction of vectors for stable transformation and expression of heterologous genes in plastids of Angiosperm plants.

### a) Obtainment of rbcL-border.

The rbcL-border fragment was obtained by using the oligonucleotides described in SEQ ID NO: 1 and SEQ ID NO: 2 for the amplification by polymerase chain reaction (PCR) of a 1524 bp DNA fragment corresponding to the gene that encodes for rbcL protein of tobacco. For the PCR was used chloroplast DNA purified from leaves of N. tabacum var. SR1. The amplified DNA fragment was cloned in pBScript SK vector (Stratagene, USA), previously digested with SpeI(Klenow)/EcoRV restriction enzymes, to produce the pBSrbcL clones. Some of the positive clones were totally sequenced and it was demonstrated that in spite of using a high fidelity DNA polymerase when performing PCR (Pfu DNA Polymerase, Promega, USA) all clones presented mutations. This fact and others evidences non-related to the present invention, indicated that rbcL protein of tobacco was toxic to *E. coli* when expressing from its own promoter. For this reason, it was decided to replace a low conserved DNA fragment of the 5' untranslated region of *rbcL* gene by a DNA fragment that contains an "ideal" lac operator allowing to clone this border fragment in lacI⁺ *E. coli* strains. To perform this mutagenesis the pBSrbcL13 clone (one which contains a mutation in the SacII/BamHI fragment of cloned *rbcL* gene) was digested with NcoI and EcoRI restriction enzymes, at positions -162 and -29 respectively, and this fragment was replaced by a synthetic DNA fragment carrying an "ideal" lac operator inserted in the -124 to -97 region of the *rbcL* gene (SEQ ID NO: 3).

Once the lac operator was inserted in pBSrbcL13 clone (called now pBSrbeL131acOp), the rbcL fragment was excised by XbaI(Klenow)/SalI restriction enzymes and it was cloned into a low copy number vector pBR322 (New England Biolabs, USA) previously digested with SalI and EcoRV restriction enzymes (XbaI site was restored), to obtain the plasmid pBR322rbcL131acOp, where the rbcL gene transcription occurs in SalI/XbaI direction.

Then, replacing the SacII/BamHI fragment of pBR322rbcL131acOp by a mutationless SacII/BamHI fragment obtained from another sequenced pBSrbcL clone, we eliminated the mutation present in the rbcL13 clone. This way it was obtained the plasmid pBR322rbcLOK, containing the rbcL-border (SEQ ID NO:4).

### b) Obtainment of atpB-border.

The atpB-border fragment was obtained by using the oligonucleotides described in SEQ ID NO: 5 and SEQ ID NO: 6 for the amplification by PCR of a 1754 bp DNA fragment corresponding to the gene that encodes for plastid atpB protein of rice. For the PCR was used DNA purified from chloroplasts of Oriza sativa var. IAC-18. The amplified DNA fragment was cloned into pBScript KS vector (Stratagene, USA) previously digested with HincII restriction enzyme (the HincII(SalI) restriction site is restored at 5' region of the cloned fragment), to produce the pBSatpB clones. Some of the positive clones were sequenced and it was chosen a totally correct clone.

The correct pBSatpB clone was digested with HindIII and SalI restriction enzymes at 5' end of the fragment (the original sequence is restored and the SalI site is lost after cloning) for cloning a synthetic DNA fragment which carries additional restriction sites and part of the 5' untranslated region of the rice *atpB* gene that contains the NEP promoter (SEQ ID NO: 7), obtaining the plasmid pBSatpBcomplete. In this vector the transcription of the *atpB* gene occurs in direction from HindIII to XhoI. The nucleotide sequence of the constructed atpB-border is shown in SEQ ID NO: 8.

### c) Obtainment of selection cassette with repeated borders.

The 1kb DNA fragment comprising the gene that encodes for hygromycin resistance (*hgh*) was obtained starting from the plasmid pBSBar, by PCR amplification using the oligonucleotides described in SEQ ID NO: 9 and SEQ ID NO: 10, which introduce a Shine-Dalgarno sequence 7 bp before the gene translation start, as well as restriction sites for facilitating the manipulation of this fragment. The amplified DNA fragment was digested with KpnI restriction enzyme and cloned into KpnI/SmaI-digested pUC19 vector (the SmaI site is conserved after cloning), giving rise to the plasmid pUC19Hyg. Subsequently, the obtained plasmid was digested with HindIII and SmaI restriction enzymes, and it was introduced a synthetic DNA fragment comprising restriction sites that will be useful for further genetic manipulations (SEQ ID NO: 11). The obtained plasmid vector was called pUC19-linker-Hyg.

The DNA fragment that will be repeated for eliminating the marker gene by homologous recombination once obtained the homoplasmic transplastomic plants, was obtained from a DNA fragment that encodes for the s10 protein of phage T7, present in the pET3xb expression vector (Novagen, USA). The pET3xb vector was initially digested with BamHI and NdeI, and the obtained 780bp DNA fragment was re-digested with TaqI(Klenow) and

KpnI, to produce a DNA fragment of approximately 310 bp that was cloned into SmaI/KpnI-digested pUC19 vector, for obtaining the plasmid pUC-Spacer.

The s10 gene fragment of 310 bp from pUC19-Spacer was cloned downstream of the *hgh* gene as an XbaI(Klenow)/KpnI fragment in the plasmid pUC19-linker-Hyg previously digested with EcoRI(Klenow)/KpnI, giving rise to pUC19-linker-Hyg-Spacer.

The second 310 bp repeated fragment was cloned upstream of the *hgh* gene, as an EcoRI(Klenow)/BamHI fragment, in the plasmid pUC19-linker-Hyg-Spacer previously digested with SmaI and BgIII restriction enzymes, to produce the plasmid pUC-linker-Spacer-Hyg-Spacer.

The bi-directional terminator rrnBT1T2 from E. *coli* was obtained from the pTrcHisB expression vector (Invitrogen. USA), by digestion with BspHI, treatment with Mung Bean nuclease and HindIII digestion, for cloning the 470 bp terminator fragment into pBScript SK vector previously digested with HindIII and SmaI restriction enzymes, to obtain the plasmid pBS-rrnBT1T2.

Finally, the DNA fragment comprising the rrnBT1T2 terminator was cloned, as a HindIII(Klenow)/XbaI fragment, downstream of the Spacer fragment that is continuous to the *hgh* gene in the plasmid pUC-linker-Spacer-Hyg-Spacer previously digested with BamHI(Klenow)/XbaI, to produce the plasmid pUC-linker-Spacer-Hyg-Spacer-rrnBT1T2. The cassette comprising from linker to rrnBT1T2 was excised form the previous construction as a HindIII/Xbal fragment, and cloned in pBScript SK vector HindIII/XbaI-digested to obtain a plasmid with the selection cassette with repeated borders, pBS-linker-Spacer-Hyg-Spacer-rrnBT1T2 (SEQ ID NO: 12).

### d) Construction of the vector for stable transformation and expression of heterologous genes in plastids of Angiosperm plants, pVTPA-f.

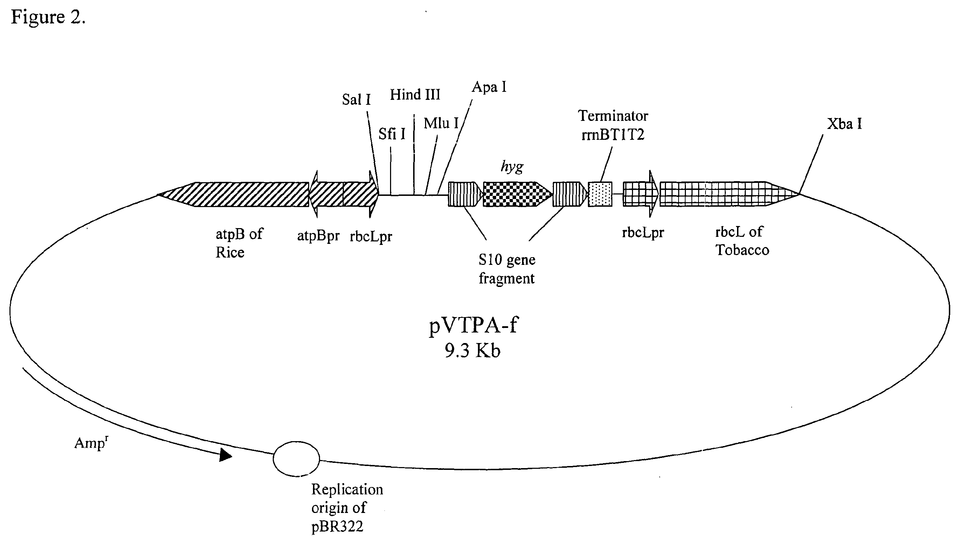

The vector for stable transformation and expression of heterologous proteins in plastids of Angiosperm plants that this invention proposes was assembled as follows: the selection cassette comprising the linker-Spacer-Hyg-Spacer-rrnBT1T2 was cloned as a NotI(Klenow)/SalI fragment in the vector pBR322 previously digested with EcoRI(Klenow)/SalI for producing the plasmid pBR322-sp-Hyg-sp-T. Subsequently, the rbcL-border from plasmid pBR332rbcLOK was cloned as a HindIII(Klenow)/XbaI fragment in the plasmid pBR322-sp-Hyg-sp-T digested with BamHI(Klenow)/XbaI, to obtain the construction pBR-sp-Hyg-sp-T-rbcL. Finally, the atpB-border from plasmid pBSatpBcomplete was cloned as a HindlII/XhoI fragment in the pBR-sp-Hyg-sp-T-rbcL digested with HindIII and SalI. So this way is obtained a vector with the structure described in Figure1-B, called pVTPA-f (see the map in Figure 2). The nucleotide sequence of a DNA fragment from the pVTPA-f, between positions +651 (SalI site) and +1 (EcoRI site) of the pBR322 carrier vector, is shown in sequence SEQ ID NO: 13.

The features of pVTPA-f vector allow the stable transformation of Angiosperm plant plastids and the obtainment of transplastomic plants that express recombinant proteins encode by genes that were cloned in this vector, whenever these genes contain ribosome binding sites at a proper distance from their translation initiation codons.

### e) Vector pVTPA: variant of the vector pVTPA-f that allows the insertion and expression in plastids of several genes as independent transcriptional units.

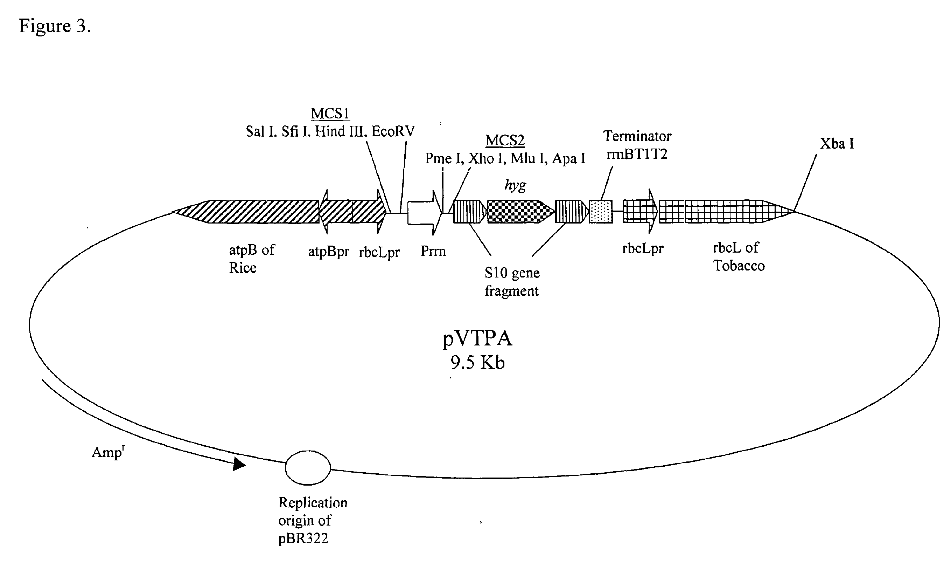

The variant of the vector pVTPA-f that allows the insertion and expression in plastids of several genes as independent transcriptional units was obtained by inserting into cloning sites of the pVTPA-f vector DNA sequences able of promoting the transcription in plastids, as well as the termination of transcription (see Figure 1-E). For this purpose as an example, a synthetic DNA fragment comprising the promoter of the 16S subunit of plastid ribosomal RNA (Prrn) (SEQ ID NO: 14), and containing cloning sites for facilitating genetic manipulations, was inserted between the EcoRI and SmaI restriction sites of the vector pBluescript SK, for obtaining the plasmid pBSPrnn. Subsequently, the DNA fragment comprising Prrn promoter was cloned as a HindIII/MluI fragment in plasmid pVTPA-f, giving rise to construction pVTPA (see Figure 3 and sequence SEQ ID NO: 15).

### Example No.2: Expression of a heterologous monocistronic unit in tobacco transplastomic plants (pVTPA-f-GUS).

For expressing an heterologous gene in transplastomic plants by using the vector pVTPA-f, it is necessary that this gene carries a ribosome binding site (RBS) approximately 5 to 15 bp upstream of its translation initiation codon. For this reason, and with the aim to facilitate the cloning of foreign genes in our vector, it was designed an intermediary vector (pVIEP) that allows to insert herein the genes of interest and later excise them by restriction digestion before cloning in the vectors pVTPA-f or pVTPA. The cloning of genes in the pVIEP vector introduces a RBS to them, and at the same time allows the addition of a transcriptional terminator or an additional promoter for the correct expression in the vector aim of this invention.

For constructing the vector pVIEP, a DNA fragment comprising the promoter of tobacco *psbA* gene with part of its 5' untranslated region deleted (between positions -68 and -25 starting from the initiation of translation) for avoiding the translation control of this gene by light (PpsbA*, SEQ ID NO: 16), was cloned as an EcoRI/SmaI fragment in the vector pBluescript SK, to obtain the plasmid pBSPpsbA*. Subsequently, a synthetic DNA fragment containing a mini-cistron (for stabilizing the mRNA and help its translation), a ribosome binding site (RBS) and multiple cloning sites (SEQ ID NO: 17) were cloned downstream of the PpsbA* promoter as a NdeI/SacI fragment (SacI site is lost after cloning) in the plasmid pBSPpsbA*, giving rise to plasmid pBSPpsbA*-linker.

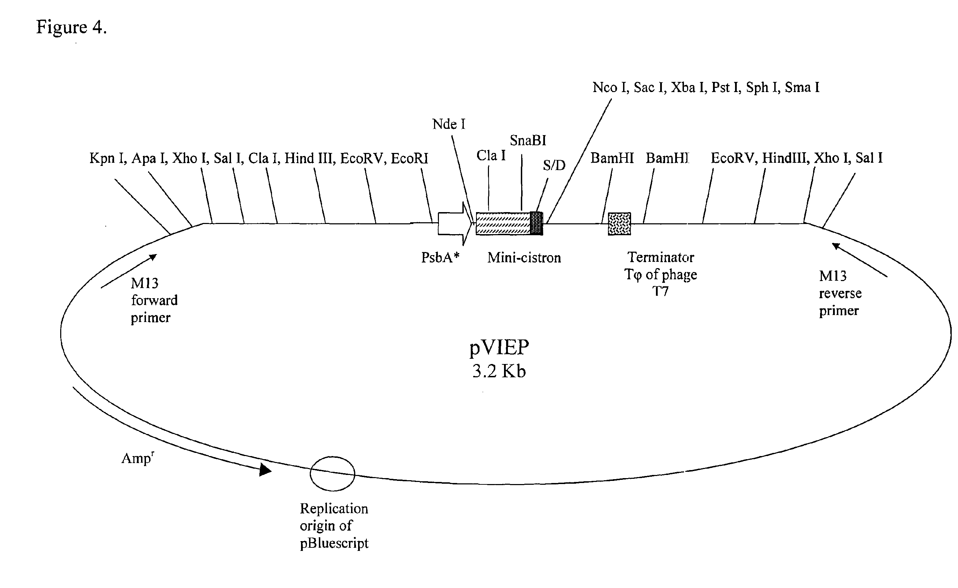

Finally, the pVIEP vector was obtained by inserting the phage T7 (Tϕ) transcription terminator, obtained from plasmid pET3c (Novagen, USA) by BamHI/EcoRV digestion, in the plasmid pBSPpsbA*-linker BamHI/StuI-digested. The sequence SEQ ID NO: 18 corresponds to the primary structure of the pVIEP vector (see Figure 4) between the KpnI and the second SalI sites.

A version of the intermediary vector pVIEP without the PpsbA* promoter and the mini-cistron was obtained by digesting pVIEP with EcoRI(Mung bean nuclease)/SnaBI, and religating, to produce the construction pVIEP-2.

For expressing an heterologous monocistronic unit in transplastomic plants, the *uidA* gene (*gus*) was obtained from plasmid pDMC200 (CAMBIA) by digesting with NcoI/SmaI, and ligated into pVIEP-2 vector NcoI/SmaI-digested to obtain the plasmid pVIEP-GUS. A DNA fragment comprising the *uidA* gene fused to the RBS obtained from the plasmid pVIEP-GUS, was cloned downstream of rice rbcL promoter as a HindIII/SmaI fragment in the plastid transformation vector pVTPA-f MluI(Klenow)/HindIII-digested, to obtain the construction pVTPA-f-GUS (SEQ ID NO: 19). Transplastomic plants transformed with the pVTPA-f-GUS vector will express the genes *hgh* and *uidA* as a bi-cistronic unit under the transcriptional control of the rbcL promoter, while the selection is maintained during transformation stages; however, the marker gene is going to be removed by homologous recombination once the transplastomic plants are cultivated in non selection conditions, and the expression of the *uida* gene will remain further from a monocistronic expression unit. The functioning of this construction was demonstrated by obtaining tobacco transplastomic plants according to the following method:
Leaves of tobacco (var. SR1) from 4-6 weeks old plants grown *in vitro* were placed abaxial side up on shoot induction medium (SI: MS salts and vitamins (Murashige T., Skoog F. Physiol. Plant. 1962, 15:493-497), BAP 1mg/L, NAA 0.1mg/L, sucrose 25g/L, phytagel 4g/L, pH 5.7) supplemented with 0.4 M of manitol for an osmotic pre-treatment of 2-4 hours. Gold particles of 0.6 µm (BioRad) were coated with DNA for bombardment as published (Russell D.R., Wallace K.M., Bathe J.H., and others. Plant Cell Rep. 1993, 12:165-169). Transformation was performed by using the PDS-1000/He biolistic gun (Biorad) with a pressure of 1100 psi, a shoot distance of 6cm and one shoot per leaf sample. Bombarded leaves were maintained on iso-osmotic medium for 14-16 hours and later were placed on SI medium for 2 days. Subsequently, leaves were cut into sections (3mm x 3mm) and transferred with abaxial side down to SI medium containing 5mg/L of Hygromycin B (Duchefa, Netherlands). Resistant green shoots and calli appear in 5-8 weeks of culture; green calli and shoots were transferred onto the same selective medium for a second and a third selection cycle using increasing concentrations of Hygromycin B (10 n:g/L and 15 mg/L). Finally, resistant shoots were rooted on MS medium (MS salts and vitamins, sucrose 30 g/L, phytagel 4 g/L, pH 5.8) supplemented with 15 mg/L of Hygromycin B to obtain plants. All of the culture stages were carried out under a regime of 16 hours of light and 8 hours of darkness.

The GUS activity of some clones resistant to Hygromycin B was analyzed by histochemical assay using X-gluc according to Jefferson (Jefferson R.A. Plant Mol. Biol. Rep. 1987, 5:387-405). The results of 3 transformation experiments and their controls are shown in Table 2.

**Table 2.**

| Obtainment of transplastomic plants with the construction pVTPA-f-GUS. | | | |
|---|---|---|---|
| Experiment. | No. of bombarded leaves. | No. of clones resistant to Hygromycin. | GUS⁺ clones. |
| I | 10 | 11 | 11/11 |
| II | 10 | 14 | 14/14 |
| III | 10 | 9 | 9/9 |
| Total | 30 | 34 | 34/34 |
| Negative controls (bombarded without plasmid) | 30 | 0 | - |
| | | | |

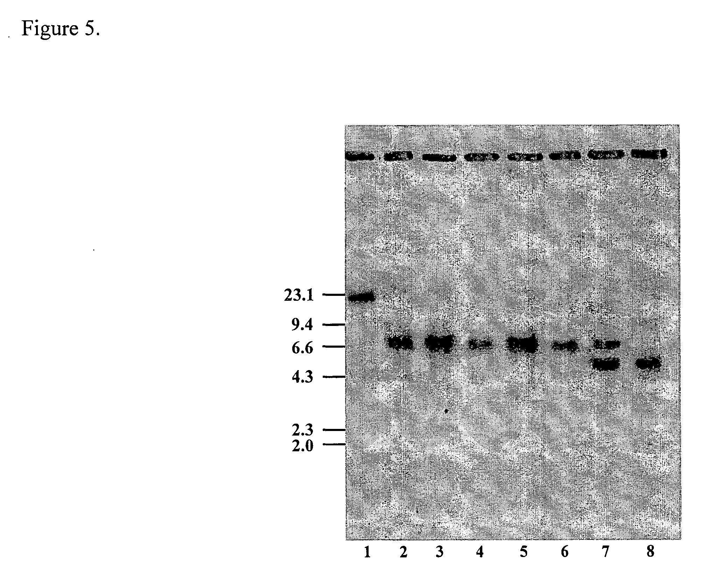

Additionally, for some clones selected at random it was demonstrated the integration of the chimeric DNA fragment into chloroplast genomes by Southern blot (Sambrook J., Fritsch E.F., Maniatis T. 1989. Molecular cloning: a laboratory manual. 2^{nd} edition. Cold Spring Habor Laboratory Press) of chloroplast DNA purified by sucrose gradient, digested with Pstl restriction enzyme and using as probe a labeled fragment of the *rbcL* gene (Figure 5). All of the analyzed positive clones yielded the expected band of approximately 6.8 kb and the negative control (non transformed plant) yielded the expected band of 21.5 kb. When some of the transplastomic clones were propagated in non selective culture medium, it was observed in the Southern blot that these clones yielded a hybridizing band of 5.5 kb, which confirms the elimination of the marker gene *(hgh)* by homologous recombination between the direct repeated sequences that border the gene.

Some rooted transplastomic clones were transferred to greenhouse for obtaining seeds by self-pollination. The collected seeds were germinated in MS medium and MS supplemented with 15 mg/L of Hygromycin B, demonstrating the elimination of marker gene in these clones, whereas the GUS activity was maintained invariable, confirming this way the homoplasmic state of transplastomic plants.

It was also noted in our experiments that unlike the results obtained when using Spectinomycin as selection agent for plastid transformation experiments, we didn't obtain spontaneous mutants when using Hygromycin B as selection agent. However, it was necessary to establish the appropriate concentrations of selection agent for recovering transplastomic plants.

These experimental data confirm the functionality of the vector aim of this invention, and its usefulness for obtaining transplastomic plants that express genes of interest from any source.

### Example No.3: Expression of a heterologous monocistronic unit in tobacco transplastomic plants under the control of two plastid functional promoters (pVTPA-aadA).

By using the pVTPA vector it is possible to express in plastids a gene under the control of

Prrn and rice rbcL promoters; for this purpose, the gene that encodes for the enzyme aminoglycoside 3'-adenylyltransferase (*aadA,* which confers resistance to the antibiotics Streptomycin and Spectinomycin) was amplified by PCR from vector pDE1001 (Department of Genetics, Gent University, Belgium) using the oligonucleotides described in sequences SEQ ID NO: 20 y SEQ ID NO: 21. The amplified DNA fragment was cloned into the vector pVTPA XhoI(Klenow)-digested under the transcriptional control of the two promoters mentioned above, giving rise to the construction pVTPA-aadA (SEQ ID NO: 22).The tobacco transplastomic plants were obtained according to the methodology described in the Example No.2, with the peculiarity that transplastomic clones were selected by using Spectinomycin (500mg/L) in the first selection cycle and Streptomycin (500mg/L) during the second selection cycle. For comparing the frequency of obtaining transplastomic plants by using vectors with different features, we carried out some transformation experiments with the vector pVSR326MOD (ICGIB, New Delhi, India).

This vector has inserted between the tobacco *rbcL* and *accD* genes two expression cassettes containing: the *gus* gene under regulation of the *psbA* gene promoter and terminator; and the *aadA* gene under the control of the Prrn promoter and the terminator of the *rbcL* gene. The results of these transformation experiments after two regeneration/selection cycles and three cycles of *in vitro* culture without selection are shown in Table 3.

**Table 3.**

| | | | | |
|---|---|---|---|---|
| Comparison of transformation frequency between vectors pVTPA-aadA and pVSr326MOD. | | | | |

| Experiment. | Genetic Construction. | No. of bombarded leaves. | No. of clones resistant to Spectinomycin. | No. of clones per bombarded leaves. |
|---|---|---|---|---|
| I | pVTPA-aadA | 10 | 19 | 1.9 |
| | pVSR32GMOD | 10 | 13 | 1.3 |
| II | pVTPA-aadA | 10 | 11 | 1.1 |
| | pVSR326MOD | 10 | 8 | 0.8 |
| III | pVTPA-aadA | 10 | 16 | 1.6 |
| | pVSR326MOD | 10 | 10 | 1.0 |
| Total | pVTPA-aadA | 30 | 46 | 1.5 ±0.4 |
| | pVSR326MOD | 30 | 31 | 1.0 ±0.3 |
| Negative controls (bombarded without plasmid) | | 20 | 4 | 0.2 |

The obtained experimental results confirm our hypothesis that, without carrying terminator sequences of plastid origin, the vector aim of this invention provides a high frequency of transplastomic plants production. Additionally, it was demonstrated the possibility that offers this vector for the expression of a foreign gene (in this case the *aadA* gene) under the transcriptional control of two promoters in tandem, PrbcL and Prrn.

Example No.4: Expression of a heterologous bi-cistronic unit in tomato transplastomic plants (pVTPA-f-GUS-aadA).

The vector aim of this invention offers the possibility of expressing several genes in form of policistronic unit. With the purpose of illustrating this possibility, in vector pVTPA-f GUS (see Example No.2) was inserted the *aadA* gene downstream of the *gus* gene to form a tri-cistronic unit (considering that the *hgh* marker gene will be also expressed as a part of the mRNA produced from the rbcL promoter), that after the elimination of the *hgh* selection marker by homologous recombination, will express only the genes that encode for the β-glucuronidase and the aminoglycoside 3'-adenylyltransferase.

The *aadA* gene was amplified by PCR as described in Example No.3, and cloned in the ApaI site (Klenow blunted) of the plasmid pVTPA-f-GUS, giving rise to the vector pVTPA-f-GUS-aadA (SEQ ID NO: 23). This new vector was used for obtaining tomato transplastomic plants according to the methods described below:
Sterilized tomato seeds (var. Campbell-28) were germinated for 12 days on MSO medium (MS salts, Gamborg B5 vitamins, sucrose 30g/L, phytoagar 8g/L, pH 5.8) half diluted, and the cotyledonal leaves were collected in liquid MSO medium and cultivated with the abaxial side down in a petri dish with solid MSO medium supplemented with 1mg/L of zeatin and 0.4 M of manitol for the osmotic pre-treatment of 4 hours. Transformation was performed by biolistic method as described in Example No.2 for tobacco. The bombarded cotyledonal leaves were maintained in the same medium for 14-16 hours and later were placed with the abaxial side up on MSO medium supplemented with 1 mg/L of zeatin for two days. Subsequently, the bombarded cotyledonal leaves were transferred with the abaxial side up to MSO medium supplemented with zeatin 1 mg/L and Hygromycin-B 5 mg/L (Duchefa, Netherlands). Resistant green shoots and calli appear in 3-6 weeks of culture and are transferred to the same selective medium for a second selection cycle with a Hygromycin-B concentration of 10 mg/L. After 3-4 weeks, the resistant green shoots and calli were transferred to MSO medium with zeatin 0.1 mg/L and Hygromycin-B 15 mg/L, and 3 weeks later were rooted on MSO medium supplemented with Hygromycin-B 15 mg/L. All the culture stages were performed with a regime of 16 hours of light and 8 hours of darkness. The resistance to 500 mg/L of Spectinomycin (Spc^{r}) as well as GUS gene expression (by histochemical assay), were evaluated in the obtained clones. The results of three transformation experiments are shown in the following table:

**Table 4.**

| Obtainment of tomato transplastomic plants with the construction pVTPA-f GUS-aadA. | | | | |
|---|---|---|---|---|
| Experiment. | No. of bombarded cotyledonal leaves. | No. of Hygromycin resistant clones. | Spc^{r} clones. | GUS⁺ clones. |
| I | 75 | 6 | 6 | 6 |
| II | 100 | 9 | 9 | 9 |
| III | 100 | 7 | 7 | 7 |
| Total | 275 | 22 | 22 | 22 |

When plants were cultured under greenhouse conditions, it was not appreciated phenotype differences among transplastomic plants and non-transformed plants of tomato, so it was confirmed the functioning of hybrids atpB and rbcL proteins, and the feasible of genetically manipulating these proteins for improving the metabolic activity of plastids. This example illustrates not only the capacity of the vector aim of this invention for expressing various genes as part of the same mRNA, but besides, confirms the universality of these vectors as vehicles for obtaining transplastomic Angiosperm plants different from those plants used as donors of the border sequences for integration of heterologous DNA into plastid genomes.

### Example No.5: Expression of two independent transcriptional units in tobacco transplastomic plants (pVTPA-HB-aadA). Obtainment of transplastomic plants that express a multimeric vaccine antigen.

The gene that encodes for the hepatitis B surface antigen *(HBsAg)* (Valenzuela P; Gray P; Quiroga M; Zaldivar J; Goodman H.M; Rutter W.J. Nature 1979, 280:815-819) was obtained from plasmid pSAO503 (CIGB, Cuba) by digestion with EcoRI(Klenow blunted)/ NcoI and inserted into NcoI/SmaI-digested pVIEP-2 vector (see Example 2) for fusing a ribosome binding site upstream of the *HBsAg* translation initiation codon, obtaining this way the plasmid pVIEP-2-HB. The DNA fragment of 820 bp comprising the gene that encodes for hepatitis B surface antigen was obtained from plasmid pVIEP-2-HB by digestion with XhoI and cloned upstream of the *aadA* gene in SalI-digested pVTPA-aadA vector (see Example No.3), to obtain the vector pVTPA-HB-aadA (SEQ ID NO: 24), that carries the *HbsAg* gene in correct orientation under the transcriptional control of the rice rbcL promoter and also expresses the marker genes *aadA* and *hgh* under the Prrn promoter.

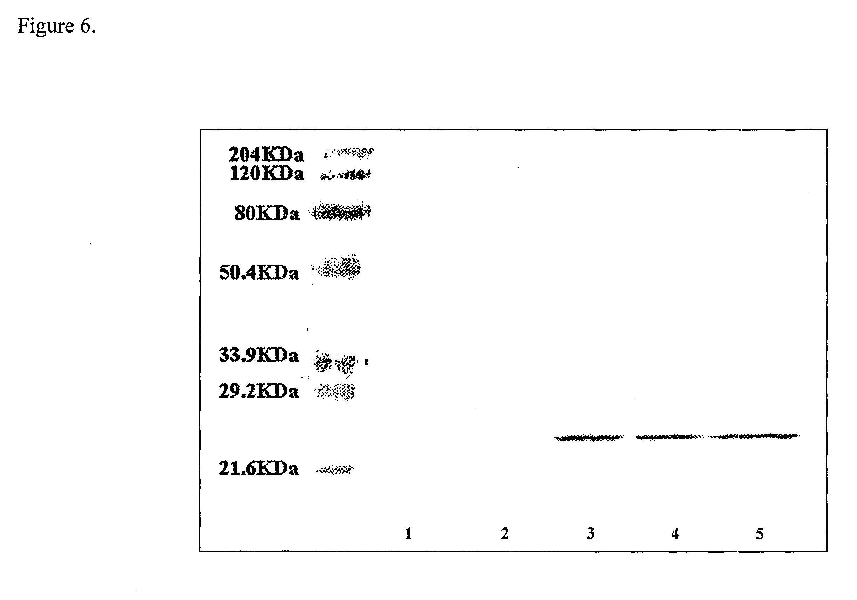

The vector pVTPA-HB-aadA was used for obtaining tobacco transplastomic plants that express the hepatitis B surface antigen, according to the transformation protocol described in Example No.2. The selection of transplastomic plants was done initially with Hygromycin-B 10 mg/L, and finally was checked the resistance to Spectinomycin 500 mg/L, and the expression of the HbsAg by an ELISA specific assay (CIGB, Cuba) and Western blot (Figure 6). The obtained results are shown in Table 5:

**Table 5.**

| Obtainment of tobacco transplastomic plants with the construction pVTPA-HB-aadA. | | | | |
|---|---|---|---|---|
| Experiment. | No.of bombarded leaves. | No. of Hygromycin resistant clones. | Spc^{r} clones. | No. of clones expressing the HBsAg (ELISA). |
| I | 10 | 13 | 13 | 9 |
| II | 10 | 11 | 11 | 7 |
| Total | 20 | 24 | 24 | 16 |

These results confirm the usefulness of the vector aim of this invention for expressing two or more genes as independent transcriptional units in the genome of transplastomic plants. Additionally, it has been confirmed the utility of this vector to express in plastids genes that encode for useful proteins that form multimeric complexes. In particular, we achieved the expression of the surface multimeric antigen of hepatitis B virus. It is obvious that what is illustrated here is applicable to other vaccine antigens, and that the same, once expressed in plant cells, can be formulated as preparations able to induce immunity against these infectious agents when provided to human being or animals. Others multimeric proteins that could be expressed by this vector are the immunoglobulins.

### Example No.6: Expression of an heterologous gene in plastids under the control of two promoters and a mini-cistron (pVTPA-GUS1-aadA and pVTPA-GUS3-aadA).

The intermediary vector for cloning and expression of genes in the vectors aim of the present invention, pVIEP (see Example No.2), allows cloning a gene under the control of PpsbA* promoter and a mini-cistron designed for increasing the stability and promoting the translation of produced mRNA. This was used for cloning the gene *uidA* as described in Example No.2 to obtain the plasmid pVIEP-GUS1.

For checking the functionality of the mini-cistron, it was made a genetic construction in which mini-cistron was eliminated from the plasmid pVIEP by digestion with SnaBI and

NdeI, blunted with an S1 nuclease treatment and re-ligated to obtain the pVIEP-3 vector; where the *uidA* gene was cloned as described previously giving rise to plasmid pVIEP-3. Both expression cassettes containing the *gus* gene were obtained from intermediary vectors by HindIII digestion and cloned into pVTPA-aadA vector. The constructions finally obtained contain the *gus* gene under the control of rice rbcL and PpsbA* promoters, and they were named pVTPA-GUS1-aadA and pVTPA-GUS3-aadA respectively. Plastids of tobacco plants were transformed with these plasmids as described in Example No.2.

Transplastomic plants were obtained with both constructions, and we made a comparison of GUS expression levels of 5 clones from each construction by the fluorimetric method using 4-MUG, according to the protocol described by Jefferson (Jefferson R.A. Plant Mol. Biol. Rep. 1987, 5: 387-405). The results are shown in Table 6.

**Table 6.**

| Expression of *uidA* gene in tobacco plastids under control of two promoters and a mini-cistron. | | | |
|---|---|---|---|
| Construction. | Clones. | GUS activity (pM 4-MU/min x µg of total proteins). | Average ± SD |
| pVTPA-GUS1-aadA | GUS1-1.3.1 | 2490 | 2475 ±903 |
| | GUS1-3.2.2 | 1736 | |
| | GUS1-9.1.2 | 4125 | |
| | GUS1-18.1.1 | 957 | |
| | GUS1-23.2.1 | 3068 | |
| pVTPA-GUS3-aadA | GUS3-2.1.3 | 517 | 978 ± 300 |
| | GUS3-7.1.1 | 1174 | |
| | GUS3-11.2.1 | 891 | |
| | GUS3-19.2.3 | 1532 | |
| | GUS3-21.1.1 | 775 | |

As the previous table shows, it was obtained high levels of GUS activity with both constructions that express the *uidA* gene under control of rice rbcL and PpsbA* promoters in tandem. Additionally, it was shown that the GUS activity average values in the transformed plants with the genetic construction that contain the mini-cistron are significantly higher, so it is demonstrated the functionality of this genetic element in plastids and its usefulness as a tool for increasing gene expression in this plant organelle.

### Example No.7: Obtainment of rice transplastomic plants with the construction pVTPA-f GUS-aadA.

Rice transplastomic plants were obtained by using the following protocol:
Rice embryogenic calli were produced from sterilized rice mature seeds cultured in N6-2 medium (Salts and vitamins of N6-2 medium (Chu C.C; and others. Scientia Sinic1975, **18**:659); myo-inositol 0.1 g/L; casein hydrolysate I g/L; 2,4D 2mg/L; sucrose 30 g/L; phytagel 3 g/L, pH 5.7) for 21 days. These calli were sub cultivated on fresh N6-2 medium for 5 days, and later were placed on N6-2 medium supplemented with 3 mg/L of kinetin for 48-72 hours in the darkness. Before bombardment embryogenic calli are sub cultivated in the same medium supplemented with manitol 0.4 M for the osmotic pre-treatment. Approximately 30 calli were placed on a petri dish and gold particles (0.6 µm) coated with plasmid pVTPA-f-GUS-aadA were used for bombardment as described in Example No.2. After bombardment calli remain in the same osmotic medium for 16 hours in the darkness and subsequently are sub cultivated in N6-2 medium for 2 days in the same conditions.

Later, calli are transferred to N6-2 medium supplemented with 20 mg/L of Hygromycin B. After 10 days, antibiotic resistant calli are transferred to the same fresh medium for a second selection cycle for 10 days. Finally, resistant calli are transferred to a modified KIBAN regeneration medium (Salts and vitamins of MS medium; kinetin 3 mg/L; BAP 0.5 mg/L; NAA I mg/L; maltose 30 g/L; phytagel 4.5 g/L; pH 5.8) supplemented with 30 mg/L of Hygromycin B, and the obtained green shoots of approximately 2 cm after 3-4 weeks of culture in a photoperiod of 16 hours of light and 8 hours of darkness, are transferred to a micro propagation cycle in modified liquid MS medium (Salts and vitamins of MS medium; sucrose 30 g/L; BAP 5 mg/L; pH 5.7) with 20 mg/L of Hygromycin B. Shoots are placed in erlenmeyers with 50 mL of this liquid medium and incubated at 28 °C with agitation of 150 rpm and a photoperiod of 16 hours of light and 8 hours of darkness. Approximately seven new shoots are obtained per each incubated shoot after 7 days of incubation, which are rooted on solid MS medium with 30 g/L of sucrose and 30 mg/L of Hygromycin B.

The obtained transplastomic clones were evaluated for Spectinomycin resistance (500 mg/L), as well as the expression of *gus* gene by histochemical assay. In addition, it was proved that the pre-treatment of the cells with cytokinins prior to bombardment significantly increased the frequency of transplastomic plants production. The results of these transformation experiments are shown in Table 7.

**Table 7.**

| Obtainment of rice transplastomic plants (var. IAC-28) with the construction pVTPA-f-GUS-aadA, with and without the pre-treatment with cytokinins (kinetin 3 mg/L). | | | | |
|---|---|---|---|---|
| Experiment. | No of bombarded plates. | No. of Hygromycin resistant clones. | Spc^{r} clones. | GUS⁺ clones. |
| I | 10 | 2 | 2 | 2 |
| II | 10 | 1 | 1 | 1 |
| III+Kinetina | 10 | 7 | 7 | 7 |
| IV + Kinetina | 10 | 8 | 8 | 8 |

The present example confirms the universality and functioning of the vectors aim of the present invention by transforming monocotyledonous species.

### Example No.8: Obtainment of sugar cane transplastomic plants with the construction pVTPA-f-GUS-aadA.

The obtainment of sugar cane transplastomic plants was performed as described below: Young sugar cane leaves of the basal part of culms from 6 month old field plants (var. Ja60-5) were cut into 0.5 x 1 cm segments, sterilized and cultivated under light on P+5 solid medium (Salts and vitamins of MS; myo-Inositol 100 mg/L; casein hydrolyzate 500mg/L; sucrose 20 g/L; 2,4D 5mg/L; phytagel 4 g/L; pH 5.7) supplemented with 3 mg/L of kinetin for 48 hours. Later, the explants were placed for 6-8 hours under light on the same medium supplemented with manitol 0.4 M for an osmotic pre-treatment before bombardment. Gold particles (0.6 µm) were coated with the plasmid pVTPA-f-GUS-aadA, and the bombardment was performed as described in Example No.2 by using the PDS-1000/He system (Bio-Rad). The bombarded explants were maintained on the same medium for 24 hours in the dark, and later were placed on P+5 medium for 2 days, following by a culture in P+5 medium supplemented with 15 mg/L of Hygromycin B in the dark. Hygromycin resistant calli appear after 4 weeks of culture and are transferred to light for regeneration on P- solid medium (P+5 medium without 2,4D) with Hygromycin B. Resistant regenerated shoots are isolated and rooted on P- medium with 20 mg/L of Hygromycin B.

The Spectinomycin (500 mg/L) resistance and the expression of *gus* gene (by histochemical assay) was evaluated in the sugarcane obtained clones. The results of two transformation experiments are shown in Table 8:

**Table 8.**

| Obtainment of sugar cane transplastomic plants with the construction pVTPA-f GUS-aadA. | | | | |
|---|---|---|---|---|
| Experiment. | No. of bombarded explants. | No. of Hygromycin resistant clones. | Spc^{r} clones. | GUS⁺ clones. |
| I | 20 | 11 | 11 | 11 |
| II | 20 | 7 | 7 | 7 |

The present example confirms again the universality of the vectors aim of this invention as vehicle for the obtainment of transplastomic Angiosperm plants different from those plants, which serve as donors for the sequences used as recombination borders for the integration of heterologous DNA into plastid genomes.

### Example No.9: Obtainment of transplastomic plants with desirable agronomic characters.

The obtainment of transplastomic plants with new incorporated agronomic characters is an important goal in the light against pests, diseases, and weeds, the increase of yield, the decrease of costs and better qualities of agricultural products, without the risk of spreading the new genes to the environment.

The *gene pat* (*bar*) of *Streptomyces hygroscopicus* (Thompson C.J; Movva N.R; Tizard R; Crameri R; Davies J.E; and others. EMBO Journal 1987, 6:2519-2523) that encodes for the phosphinothricin (active ingredient of BASTA^{TM} herbicide) acetyl transferase, was obtained from plasmid pBS-Bar (C.LG.B) by digestion with XbaI(S1 nuclease blunted) and BamHI, and was cloned into vector pVIEP-2 (see Example No.2) treated with NcoI (S1 nuclease blunted) and BamHI-digested, to produce the plasmid pVIEP2-Bar. The 670 bp DNA fragment comprising the *pat* gene with a RBS obtained from pVIEP2-Bar by XhoI digestion was cloned into pVTPA vector to produce the construction pVTPA-Bar (SEQ ID NO: 25). Tobacco transplastomic plants were obtained according to the protocol described in Example No.2, identifying the transformants by resistance to phosphinothricin (Duchefa, Netherlands) at 5 mg/L in the culture medium. The obtained results are shown in Table 9.

**Table 9.**

| Obtainment of tobacco transplastomic plants resistant to herbicide with the construction pVTPA-Bar. | | |
|---|---|---|
| Experiment. | No. of bombarded leaves. | No. of phosphinothricin resistant clones (5mg/L). |
| I | 10 | 13 |
| II | 10 | 14 |
| Control (transformed without plasmid) | 10 | 0 |

It is obvious that the cloning and expression in the vectors aim of this invention of other genes that confer resistance to herbicides, as for example, glyphosate, asulam, sulphonilurea, imidazolinone and bromoxinil, will produce transplastomic plants resistant to these chemicals. Equally, it is obvious that with the use of the vector aim of this invention is feasible to clone and express in transplastomic plants genes that confer resistance to pests and diseases, as for example the genes that encodes for the protein crystals of *Bacillus thuringiensis;* or for proteins with anti-fungal activity like chitinases and glucanases: or with anti-abiotic stress properties like choline oxidase. The modification or introduction of genes that increase the efficiency of photosynthesis, or the quality of plant products, are some of the plant functions that can be improved with the use of the universal vector for Angiosperm plastid transformation that this invention proposes.

### Example No.10: Expression of proteins of non-agronomic use in plastids.

In the Example No.5 we demonstrated the utility of the vector aim of this invention for stable introduction and expression of genes that encodes for multimeric proteins in Angiosperm plant plastids. Others proteins of medical, veterinary, or industrial use are equally feasible of being produced in transplastomic plants by means of our transformation/expression vector system.

For expressing in chloroplasts the fibrinolitic agent streptokinase, very useful for the treatment of thrombosis and heart attacks, the 1254 bp DNA fragment that encodes for the mature protein SKC-2 of *Streptococus* was excised from the pEKG-3 vector (Estrada M.P; Hernández L; Pérez A; Rodríguez P; Serrano R; Rubiera R; Pedraza A; and others. Bio/Technology 1992, 10:1138-1142) by EcoRI(S1 nuclease blunted) and BamHI digestion, and inserted into the vector pVIEP (see Example No.2) NcoI(S1 nuclease blunted)/BamHI-digested, to obtain the plasmid pVIEP-Strep. The cassette for the expression of streptokinase gene in plastids under the control of the PpsbA* promoter and the mini-cistron, was obtained by SaII/XhoI digestion from the plasmid pVIEP-Strep, and was cloned into the pVTPA vector (see Example No.1) SaII/XhoI-digested to construct the plasmid pVTPA-Strep (SEQ ID NO: 26).

Plastids of tobacco plants were transformed with the vector pVTPA-Strep as described in Example No.2. The results obtained are shown in Table 10 (the fibrinolitic activity of streptokinase produced in some clones was determined by the casein/plasminogen plate technique (Estrada M.P; Hernindez L; Pérez A; Rodriguez P; Serrano R; Rubiera R; Pedraza A; y otros. Bio/Technology 1992, **10**:1138-1142).

**Table 10.**

| Expresion of Streptokinase in tobacco plastids. | | | |
|---|---|---|---|
| No.of bombarded leaves. | No. of Hygromycin resistant clones. | Clones. | Fibrinolitic activity (UI/mg of proteins). |
| 10 | 14 | Strep2.3.1 | 650 |
| | | Strep4.5.2 | 400 |
| | | Strep5.2.2 | 1500 |
| | | Strep9.2.2 | 200 |
| | | Strep12.3.1 | 350 |
| | | Negative control | 0 |

These results confirm the utility of the vectors aim of this invention for expressing genes that encode for useful proteins in plastids, and in particular proteins of pharmaceutical or veterinary use like thrombolytics, interferons, cytokines, growth factors, hormones, antigens, cell receptors and others. It is obvious that what have been exemplified here can be also applied to the expression in plastids of others kind of enzymes and proteins of industrial use like lipases, proteases, etc by using the transformation/expression vectors described in the present document.

### Example No.11: Expression of immunoglobulins in plastids.

The DNA fragments that encode for the heavy and light chains of the monoclonal antibody CB-Hep.1 specific for HbsAg were obtained by NcoI/XbaI digestion from plasmids pHCSΩHBsAgCL and pHESΩHBsAgCH (Nadia Ramírez, "Transgenic tobacco *(Nicotiana tabacum* L.): a system for production of an antibody anti-HBsAg and its fragments", PhD. Thesis, C.LG.B., 2002) respectively, and inserted in the pVIEP vector NcoI/XbaI-digested for producing the plasmids pVIEP-Hc and pVIEP-Lc. Subsequently, the DNA fragment that encodes for the light chain was excised from pVIEP-Lc plasmid by SnaBI/BamHI digestion and inserted into pVIEP-Hc plasmid SmaI/BamHI-digested, giving rise to the bi-cistronic construction pVIEP-HcLc. Further, the expression cassette comprising the heavy and light chains, each one fused to a RBS, was cloned into pVTPA-f vector as a HindIII fragment; so, it was obtained the plasmid pVTPA-f-HcLc. This plasmid was used for the transformation of tobacco plastids as described in Example No.2.

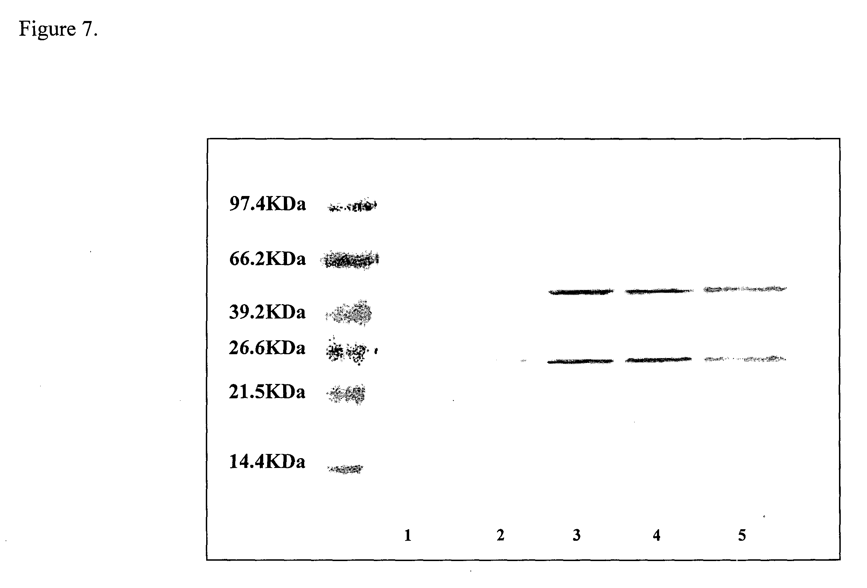

The obtained transplastomic plants were analyzed by Western blot for detecting the presence of both chains of the CB-Hep.1 monoclonal antibody (Figure 7); and additionally it was proved the functioning of the produced recombinant immunoglobulins by using protein extracts from transplastomic plants to evidence the presence of the HbsAg in an ELISA that was revealed with an rabbit anti-mouse policlonal antibody conjugated to alkaline phosphatase.

This experiment demonstrated the possibility to produce completely functional antibodies in transplastomic plants by usisng our transformation/expression vectors, and at the same time we re-affirmed the possibility to express and correctly assemble multimeric proteins in plastids.

## Claims

1. A DNA vector useful for stable transformation and expression of genes in plastids, where the gene to be expressed is inserted an the artificial intergenic region constituted by the combination of two 5' untranslated regions of plastid genes whose transcription naturally occurs in opposite directions and that belong to plants of different Classes or Divisions.

2. A DNA vector as claimed in claim 1, useful for achieving a high frequency of stable transformation and expression of genes in plastids of Angiosperm plants, with the following features:
a) contains an artificial intergenic region where it is possible to insert DNA sequences that encode for a polypeptide, under appropriated signals for its transcription and translation in plastids using transcription terminator sequences of non-plastid origin,
b) the artificial intergenic region is flanked by two genes whose transcription naturally occurs in opposite directions in plastid genomes, and whose nucleotide sequences are identical to the encoding sequences of *atpB* and *rbcL* genes belonging to plastids of Angiosperm plants from different Classes (dicotyledonous and monocotyledonous),
c) the artificial intergenic region is composed by: 5' regulatory sequences for transcription and translation of the *atpB* and *rbcL* genes; restriction sites for inserting genes of interest followed by a transcription terminator of non-plastid origin; and a second 5' regulatory region for the transcription and translation of an *rbcL* gene belonging to Angiosperm plastids from plants of a Class different to the Class of the previous *rbcL* gene.

3. A DNA vector as claimed in claim 2, comprising a border DNA sequence homologous to the sequence of plastid *rbcL* gene from tobacco or rice.

4. A DNA vector as claimed in claim 3, wherein said DNA sequence homologous to the *rbcL* gene comprises at least a fragment of the nucleotide sequence from position -291 to position +1233, starting from the translation initiation codon, of the tobacco *rbcL* gene.

5. A DNA vector as claimed in claim 4, wherein said border sequence homologous to the *rbcL* gene comprises the sequence SEQ.ID.NO: 4.

6. A DNA vector as claimed in claim 2, comprising a border DNA sequence homologous to the sequence of plastid *atpB* gene from rice or tobacco.

7. A DNA vector as claimed in claim 6, wherein said DNA sequence homologous to the *atpB* gene comprises at least a fragment of the nucleotide sequence from position -654 to position +1211, starting from the translation initiation codon, of the rice *atpB* gene.

8. A DNA vector as claimed in claim 7, wherein said border sequence homologous to the *atpB* gene comprises the sequence SEQ:ID.NO: 8.

9. A DNA vector as claimed in claim 2, wherein said transcription terminator of non-plastid origin present in the artificial intergenic region, is the bi-directional terminator rrnBT1T2 from *Escherichia coli* or an homologous thereof.

10. A DNA vector as claimed in claim 2, having inserted in the artificial intergenic region a gene that allows the selection oftransplastomic plants.

11. A DNA vector as claimed in claim 10, wherein said selection gene can be eliminated by homologous recombination between direct repeated DNA sequences that border the gene.

12. A DNA vector as claimed in claim 11, wherein said selection gene encodes for a protein that allows plant cells to survive in presence of an antimicrobial compound.

13. A DNA vector as claimed in claim 12, wherein said antimicrobial compound belongs to the group of aminoglycoside antibiotics.

14. A DNA vector as claimed in claim 13, wherein said antibiotic compound is the Hygromycin B.

15. A DNA vector as claimed in claim 13, wherein said antibiotic compound is the Spectinomycin.

16. A DNA vector as claimed in claim 12, wherein said antimicrobial compound belongs to the group of sulfonamides.

17. A DNA vector as claimed in claim 16, wherein said antimicrobial compound is the Sulfadiazine.

18. A DNA vector as claimed in claim 10, wherein said selection gene encodes for a protein that allows plant cells to survive in presence of an herbicide compound.

19. A DNA vector as claimed in claim 18, wherein said herbicide compound belongs to the group of glutamine synthetase inhibitors.

20. A DNA vector as claimed in claim 19, wherein said herbicide compound is the Glufosinate (phosphinothricin).

21. A DNA vector as claimed in claim 19, wherein said herbicide compound is the Bialaphos.

22. A DNA vector as claimed in claim 18, wherein said herbicide compound belongs to the group of 5-enolpiruvil-shikimate-3-phosphate synthase (EPSPS) inhibitors.

23. A DNA vector as claimed in claim 22, wherein said herbicide compound is the Glyphosate (N-phosphomethyl glycine).

24. A DNA vector as claimed in claim 18, wherein said herbicide compound belongs to the group of inhibitors of folate synthesis.

25. A DNA vector as claimed in claim 24, wherein said herbicide compound is the Asulam (methyl- (4-aminobencenosulphonyl)-carbamate).

26. A DNA vector as claimed in claim 18, wherein said herbicide compound belongs to the group of inhibitors of acetolactate synthase (ALS).

27. A DNA vector as claimed in claim 26, wherein said herbicide compound is a sulphonylurea.

28. A DNA vector as claimed in claim 26, wherein said herbicide compound is a imidazolinone.

29. A DNA vector as claimed in claim 18, wherein said herbicide compound belongs to the group of inhibitors of photosystem II.

30. A DNA vector as claimed in claim 29, wherein said herbicide compound is the Bromoxinyl (3,5-dibromo-4-hydroxibenzonitrile).

31. A DNA vector as claimed in claim 10, wherein said selection gene encodes for a protein that allows plant cells to survive in presence of a toxic compound.

32. A DNA vector as claimed in claim 31, wherein said toxic compound is the betaine aldehyde.

33. A DNA vector as claimed in claims 1 to 8, having the general structure described in Figure1-A or variants thereof.

34. A DNA vector as claimed in claims 1 to 11, having the general structure described in Figure 1-B or variants thereof.

35. A DNA vector as claimed in claims 1 to 11, having the general structure described in Figure 1-C or variants thereof.

36. A DNA vector as claimed in claims 1 to 11, having the general structure described in Figure 1-D or variants thereof.

37. A DNA vector as claimed in claims 1 to 11, having the general structure described in Figure 1-E or variants thereof.

38. A DNA vector as claimed in claims 1 to 11, having the general structure described in Figure 1-F or variants thereof.

39. A DNA vector as claimed in claim 2, having in the second 5' untranslated region of *rbcL* gene a sequence functionally homologous to the binding site of the *Escherichia coli* lacI repressor.

40. A DNA vector having upstream of the translation initiation codon of *rbcL* gene a DNA sequence that allows to repress the transcription of this gene in non induction conditions, in order to provide the maintenance and multiplication of the vector in any host.

41. A DNA vector as claimed in claim 40, having in the 5' untranslated region *of rbcL* gene a sequence functionally homologous to the binding site of the *Escherichia coli* lacI repressor.

42. A DNA vector as claimed in claim 41, where the nucleotide sequence of said lacI binding site, corresponds at least in part to the sequence described in SEQ.ID.NO: 3.

43. A DNA vector as claimed in claims 1 to 11, having inserted in the artificial intergenic region one or more monocistronic or policistronic transcriptional units under one or more plastid functional promoters.

44. A DNA vector as claimed in claims 12 to 17, having inserted in the artificial intergenic region one or more monocistronic or policistronic transcriptional units under one or more plastid functional promoters.

45. A DNA vector as claimed in claims 18 to 38, having inserted in the artificial intergenic region one or more monocistronic or policistronic transcriptional units under one or more plastid functional promoters.

46. A DNA vector as claimed in claims 43, 44 or 45, wherein at least one of the said plastid functional promoters is an rbcL promoter of the artificial intergenic region.

47. A DNA vector as claimed in claim 46, wherein the plastid functional promoters is the rice rbcL promoter comprised in sequence SEQ.ID.NO: 8.

48. A DNA vector as claimed in claims 43, 44 or 45, wherein at least one of the said plastid functional promoters is the psbA* promoter comprised in the sequence SEQ.ID.NO: 16 or variants thereof.

49. A DNA vector as claimed in claim 48, having a mini-cistron functionally linked to said psbA* promoter.

50. A DNA vector as claimed in claim 49, wherein said mini-cistron have a nucleotide sequence homologous to sequence described in SEQ.ID.NO: 17 or variants thereof.

51. A DNA vector for stable introduction and expression of genes in plastids that uses a mini-cistron for increasing the expression of genes introduced into transplastomic plants.

52. A DNA vector as claimed in claims 43, 44 or 45, wherein at least one gene of agricultural, veterinary, pharmaceutical, food or industrial interest is a component of the said transcriptional units.

53. A DNA vector as claimed in claim 52, wherein said gene of agricultural interest encodes for a protein with insecticide activity.

54. A DNA vector as claimed in claim 53, wherein said insecticide protein belongs to the family of Cry proteins from *Bacillus thuringiensis.*

55. A DNA vector as claimed in claim 53, wherein said insecticide protein is a protease inhibitor.

56. A DNA vector as claimed in claim 52, wherein said gene of agricultural interest encodes for a polypeptide with antimicrobial activity.

57. A DNA vector as claimed in claim 56, wherein said polypeptide belongs to one of the groups of the plant proteins related to pathogenicity (PR-proteins).

58. A DNA vector as claimed in claim 57, wherein said PR-protein is a glucanase.

59. A DNA vector as claimed in claim 57, wherein said PR-protein is a chitinase.

60. A DNA vector as claimed in claim 57, wherein said PR-protein is a thaumatin-like protein.

61. A DNA vector as claimed in claim 56, wherein said polypeptide is a ribosome inactivation protein (RIP).

62. A DNA vector as claimed in claim 52, wherein said gene of agricultural interest encodes for a protein that confers resistance to abiotic stress.

63. A DNA vector as claimed in claim 62, wherein said protein has choline oxidase activity.

64. A DNA vector as claimed in claim 52, wherein said gene of agricultural interest encodes for a protein that contributes to increase plant productivity.

65. A DNA vector as claimed in claim 64, wherein said protein is involved in the improvement of photosynthetic performance of the plant.

66. A DNA vector as claimed in claim 65, wherein said protein has fructose-1, 6-bisphosphate phosphatase (FBPase) activity.

67. A DNA vector as claimed in claim 65, wherein said protein has protoporphyrinogen oxidase (PROTOX) activity.

68. A DNA vector as claimed in claim 65, wherein said protein has ribulose-1-5-bisphosphate carboxylase/oxygenase (RUBISCO) activity.

69. A DNA vector as claimed in claim 65, wherein said protein takes part directly or indirectly in the carbon fixation process of the plants.

70. A DNA vector as claimed in claim 52, wherein said gene of agricultural interest encodes for a protein that contributes to increase the post-harvest conservation of the plant products.

71. A DNA vector as claimed in claim 52, wherein said gene of food interest encodes for a protein that contributes to improve the nutritional quality of the plant products.

72. A DNA vector as claimed in claim 52, wherein said gene of veterinary or pharmaceutical interest encodes for a cytokine.

73. A DNA vector as claimed in claim 72, where said cytokine belongs to the family of interferons.

74. A DNA vector as claimed in claim 72, where said cytokine belongs to the family of interleukines.

75. A DNA vector as claimed in claim 52, wherein said gene of veterinary or pharmaceutical interest encodes for a polypeptide with regulatory activity on the immune response.

76. A DNA vector as claimed in claim 52, wherein said gene of veterinary or pharmaceutical interest encodes for a polypeptide with hormonal activity.

77. A DNA vector as claimed in claim 76, wherein said polypeptide with hormonal activity is the insulin.

78. A DNA vector as claimed in claim 76, wherein said polypeptide with hormonal activity is a growth hormone.

79. A DNA vector as claimed in claim 76, wherein said polypeptide with hormonal activity is a somatotropic hormone.

80. A DNA vector as claimed in claim 76, wherein said polypeptide with hormonal activity is a gonadotropic hormone.

81. A DNA vector as claimed in claim 52, wherein said gene of veterinary or pharmaceutical interest encodes for a cell proliferation factor.

82. A DNA vector as claimed in claim 81, wherein said cell proliferation factor is the epidermic growth factor (EGF).

83. A DNA vector as claimed in claim 52, wherein said gene of veterinary or pharmaceutical interest encodes for a polypeptide with hematopoietic activity.

84. A DNA vector as claimed in claim 52, wherein said gene of veterinary or pharmaceutical interest encodes for a cell receptor.

85. A DNA vector as claimed in claim 52, wherein said gene of veterinary or pharmaceutical interest encodes for a protease inhibitor.

86. A DNA vector as claimed in claim 52, wherein said gene of veterinary or pharmaceutical interest encodes for a polypeptide with trombolytic activity.

87. A DNA vector as claimed in claim 86, wherein said polypeptide is the streptokinase.

88. A DNA vector as claimed in claim 86, wherein said polypeptide is the tissular plasminogen activator (t-PA).

89. A DNA vector as claimed in claim 52, wherein said gene of veterinary or pharmaceutical interest encodes for a vaccine antigen.

90. A DNA vector as claimed in claim 89, wherein said vaccine antigen belongs to a virus.

91. A DNA vector as claimed in claim 90, wherein said vaccine antigen belongs to hepatitis virus.

92. A DNA vector as claimed in claim 91, wherein said vaccine antigen is the hepatitis B surface antigen.

93. A DNA vector as claimed in claim 91, wherein said vaccine antigen belongs to the hepatitis A virus.

94. A DNA vector as claimed in claim 91, wherein said vaccine antigen belongs to the hepatitis C virus.

95. A DNA vector as claimed in claim 90, wherein said vaccine antigen belongs to the foot and mouth disease virus (FMDV).

96. A DNA vector as claimed in claim 90, wherein said vaccine antigen belongs to the human immunodeficiency virus (HIV).

97. A DNA vector as claimed in claim 89, wherein said vaccine antigen belongs to a bacterium.

98. A DNA vector as claimed in claim 89, wherein said vaccine antigen belongs to a protozoan.

99. A DNA vector as claimed in claim 52, wherein said gene encodes for a fragment of the variable region of an immunoglobulin.

100. A DNA vector as claimed in claim 52, wherein said gene encodes for a multimeric protein.

101. A DNA vector as claimed in claim 100, wherein said multimeric protein is an immunoglobulin.

102. A DNA vector as claimed in claim 100, wherein said multimeric protein is a hormone.

103. A DNA vector as claimed in claim 100, wherein said multimeric protein is a vaccine antigen.

104. A DNA vector as claimed in claim 100, wherein said multimeric protein is an enzyme.

105. A DNA vector as claimed in claim 100, wherein said multimeric protein is a cell receptor.

106. A DNA vector as claimed in claim 52, wherein said gene of industrial interest encodes for a protein component of a biopolymer.

107. A DNA vector as claimed in claim 52, wherein said gene of industrial interest encodes for an enzyme.

108. A DNA vector as claimed in claim 107, wherein said enzyme is a protease.

109. A DNA vector as claimed in claim 107, wherein said enzyme is a lipase.

110. A DNA vector as claimed in claim 107, wherein said enzyme is an isomerase.

111. A DNA vector as claimed in claim 107, wherein said enzyme has glycosil-hydrolase activity.

112. A DNA vector as claimed in claim 111, wherein said enzyme with glycosil-hydrolase activity is a levansucrase.

113. A DNA vector as claimed in claim 111, wherein said enzyme with glycosil-hydrolase activity is an invertase.

114. A DNA vector as claimed in claim 111, wherein said enzyme with glycosil-hydrolase activity is a levanase.

115. A DNA vector as claimed in claim 111, wherein said enzyme with glycosil-hydrolase activity is a dextranase.

116. The culture of vegetable cells in presence of cytokinins, before introducing into them any of the DNA vectors claimed in claims 1 to 115, to increase the frequency of production of transplastomic plants.

117. The use of kinetin as claimed in claim 116.

118. The Angiosperm transplastomic plants stably transformed with any of the DNA vectors claimed in claims 1 to 115.

119. The progeny oftransplastomic plants of claim 118.

120. The transplastomic plants of claims 118 and 119 that stably express at least one of the genes present in the DNA vector used for transformation.

121. The transplastomic plants of claims 118 and 119 with hybrid atpB and/or rbcL proteins.

122. The culture of transplastomic plants of claim 120.

123. The culture of cells of the transplastomic plants of claim 120.

124. The purification and use of the protein, or proteins, that produce the cells of the transplastomic plants of claim 120 as a result of the expression of said gene or genes.

125. The transplastomic plants of claims 118 to 121 that are Angiosperms.

126. The transplastomic plants of claim 125 that are dicotyledonous.

127. The transplastomic plants of claim 126 that are solanaceous.

128. The transplastomic plants of claim 127 that belong to one of the following species: tobacco, tomato or potato.

129. The transplastomic plants of claim 125 that are monocotyledonous.

130. The transplastomic plants of claim 129 that are gramineous.

131. The transplastomic plants of claim 130 that belong to one of the following species: rice, sugar cane, maize, wheat or barley.
